# EUROPEAN PATENT SPECIFICATION

(11) **EP 1 503 771 B1**
(45) Date of publication and mention of the grant of the patent: **23.07.2008**
(21) Application number: 03701586.4
(22) Date of filing: 24.01.2003
(51) Int. Cl.: A61K 36/84, A61P 25/00

(54) **SEDATIVE MATERIALS AND TREATMENTS**
SEDATIVE STOFFE UND BEHANDLUNGEN
SUBSTANCES ET TRAITEMENTS SÉDATIFS

(30) Priority: 25.01.2002 GB 0201669; 01.08.2002 GB 0217792
(43) Date of publication of application: 09.02.2005
(73) Proprietor: University of Strathclyde, Glasgow G1 1XQ (GB)
(72) Inventor: MARDER, Nora, Mariel, Ciudad Autonoma de Buenos Aires (AR); MEDINA, Jorge, Horacio, Provincia de Buenos Aires (AR); PALADINI, Alejandro, Constantino, Ciudad Autonoma de Buenos Aires (AR); VIOLA, Haydee, Ana, Maria, Provincia de Buenos Aires (AR); WASOWSKI, Cristina, Lucia, Natalia, Ciudad Autonoma de Buenos Aires (AR)
(74) Representative: Chapman, Paul Gilmour
(86) International application number: PCT/GB2003/000260
(87) International publication number: WO 2003/061678

(56) References cited:
- WO-A-01/07063
- DE-A- 3 112 737
- US-A- 3 422 090
- WASOWSKI CRISTINA ET AL: "Isolation and identification of 6-methylapigenin, a competitive ligand for the brain GABAA receptors, from Valeriana wallichii." PLANTA MEDICA, vol. 68, no. 10, 20 October 2002 (2002-10-20), pages 934-936, XP008018439 ISSN: 0032-0943

## Description

### FIELD OF THE INVENTION

The present invention relates to sedative, sleep inducing and/or anxiolytic materials and treatments and more particularly to novel extracts, fractions and substances from *Valeriana* species roots and rhizomes and the use thereof in sedation, sleep and/or anxiolysis. The present invention also relates to the potentiating effect of 6-methylapigenin and valerenic acid, the sedative activity of hesperidin and linarin and the methods to isolate 6-methylapigenin, hesperidin and linarin.

### BACKGROUND OF THE INVENTION

The roots and rhizomes of several valerian and other related plants of the genus *Valerianaceae* have been the source of a useful natural sedative known and recorded by Galen, Dioscorides and Pliny the Elder but already included in the Ayurvedic and Chinese medicines, from 800 years B.C. to 1000 years A.D.

In modern times the highly respected medicinal properties of valerian are attested by its citation in more than 22 pharmacopoeias and extensive use in Europe, Asia and several other regions of the world.

The molecular reasons for these properties, however, have remained elusive to the present day in spite of the many studies performed in the last 100 years, or so. Scores of compounds have been identified in a variety of species of the Valerianaceae. However, not a single class of molecules, let alone a particular one, could be proved to be fully responsible for the sedative properties of the drug. The generally accepted view in the art concerning the molecular reasons for the recorded sedative activity of valerian has been summarized by Houghton in 1988 as: "The sedative effect of valerian seems to be caused by a combination of depression of some CNS centers and direct relaxation of smooth muscle. The biochemical basis of this activity is still largely unknown. The use of purified and standardized extracts of valepotriates over the last 20 years has largely replaced the traditional tincture in Western Europe. The cytotoxicity of these compounds gives cause for concern and in future their absence from pharmaceuticals derived from crude drugs might be a requirement." It is generally known at present that the use of these purified valepotriate pharmaceutical forms has been abandoned altogether. Reviewing the state of the art in 1999, Houghton again reached the same conclusions (Houghton, P. J., 1988. "The biological activity of valerian and related plants". J. Ethnopharmacol., 22: 121-142; Houghton, P. J., 1999. "The scientific basis for the reputed activity of Valerian." J. Pharm. Pharmacol. 51: 505-512).

A further problem with the various known Valerian extracts is that of a lack of standardization in relation to the activity or potency of the extracts, which is of course exacerbated by the fact that to date there has still been no positive identification of the active principle(s) of Valerian.

### SUMMARY OF THE INVENTION

It is an object of the present invention to avoid or minimize one or more of the above disadvantages.

It has now been found that an active component capable of inducing sedation in mammals is obtainable from *valeriana* roots by a process comprising the steps of:
(a) 70% v/v aqueous ethanol extraction of comminuted valerian roots;
(b) 50% v/v aqueous ethyl ether extraction of the ethanolic extract after removal of the ethanol to produce an ether extract first portion and a remaining aqueous portion;
(c) dissolving in a polar and/or non-polar solvent the ether extract first portion after removal of the ether;
(d) fractionating by chromatography the dissolved first portion by stepwise elution with one or more polar and/or non-polar solvents over a suitable solid phase; and
(e) recovering various fractions or taking the remaining aqueous fraction and testing for sedation and/or benzodiazepine binding site (BDZ-bs) activity.

Sedation is understood to mean the normalisation of arousal or altertness of a subject without affecting its emotions or its stress. Sedation may involve reduction of consciousness and general locomotor activity. The present invention utilises the animal model of the hole-board assay which measures a decrease in the exploratory drive in mice and correlates this with a sedative effect.

Anti-anxiety as discussed in the present application is based on the fear that mice have for open spaces i.e. the elevated plus-maze assay.

Hypnosis may be defined as the ability to put the mice to sleep.

After removal of the ether in step (c), the ether extract first portion may be dissolved in methanol prior to fractionation.

Step (d) may comprise the step of fractionating the dissolved first portion in a 10 to 80 %v/v step-wise concentration gradient of aqueous acetonitrile using a C18 Vydac (Trade Name) reverse solid phase column, being followed by step (f) comprising recovering a 40-50 %v/v aqueous acetonitrile fraction displaying sedation activity.

Step (e) may comprise in vivo and/or vitro testing of the sedation activity of the various fractions and/or remaining aqueous portion.

The novel fraction obtainable by the above process has been found to have particularly high levels of activity in relation to both in vivo sedation activity and in vitro benzodiazepine receptor or binding site (BDZ-bs) binding activity. In particular, the sedation activity has been found to be of a much higher order than that found with any other previously known valerian extract. The active component also has comparable levels of greater activity than other conventional preparations such as those based on valepotriate mixtures, baldrinal, valerenic acid, and valeranone, whilst being substantially free of undesirable side effects such as those associated with some of these known preparations. The amounts of material recovered in the fractions comprising the active component of the present invention moreover represent a 500 fold purification relative to the dry root material and a 100 fold purification relative to a generally conventional 70% v/v aqueous ethanol extract of the dry root material.

In another aspect, the method of the present invention provides an active sedative component comprising 6-methylapigenin. The 6-methylapigenin is present in the ethyl ether extract first portion. Alternatively, and/or additionally a proportion of the 6-methylapigenin or glycosyl derivatives of 6-methylapigenin may also be present in the remaining aqueous portion.

In a yet further aspect, the present invention there provides for the use of 6-methylapigenin in the manufacture of a medicament for the preparation of a sedative for mammals.

-In a further aspect of the present invention there is provided an active component capable of inducing sedation in mammals and obtainable from valerian roots by a process of the present invention, said component having a sedative activity corresponding to at least 10 times, preferably at least 20 times, advantageously at least 50 times, most preferably at least 100 times, most desirably at least 200 times, greater than that of whole root on a weight for weight basis. In yet another aspect the present invention provides an active component capable of inducing sedation in mammals and obtainable from valerian roots by a process of the present invention, said component having a sedative activity corresponding to at least 10 times, preferably at least 30 times, advantageously at least 50 times, greater than that of a 70% v/v aqueous ethanol extract.

Preferably, the active component of the invention is substantially free of at least one of the following compounds valepotriates, baldrinals, and valerenic acid, and is desirably substantially free of all of these. Most preferably the active component of the invention has not more than 3% w/w, advantageously not more than 1% w/w, of these substances.

It has now also been found that novel active components capable of inducing sedation, sleep and/or anxiolysis in mammals are obtainable from *valeriana* roots by a process comprising the steps of:
(a) about 70% v/v aqueous ethanol extraction of comminuted valerian roots;
(b) petroleum ether extraction of the aqueous solution remaining after the elimination of the ethanol by evaporation; the petroleum ether extract is then discarded;
(c) about 50% v/v aqueous ethyl ether extraction of the aqueous solution remaining in (b) after removal of the petroleum ether by evaporation, to produce an ethyl ether extract first portion and a remaining aqueous portion;
(d) dissolving in a polar and/or non-polar solvent the dry ether extract first portion after removal of the ether;
(e) fractionating by chromatography the dissolved ether extract first portion by stepwise elution with one or more polar and/or non-polar solvents over a suitable solid phase, and recovering various fractions to obtain a novel crystalline active material;
(f) extracting the aqueous phase remaining after the ethyl ether extraction with amyl alcohol and chromatographing this extract in a column eluted with chloroform/methanol;
(g) the fractions containing the active material in (f), detected by a sleep inducing assay, are pooled and partially evaporated to obtain a second novel crystalline active material.

Step (e) may comprise fractionating the dissolved ether extract first portion in a 10 to 80% v/v step-wise concentration gradient of aqueous acetonitrile using a C18 Vydac (Trade Name) (The Separation Group Hesperia, CA, USA) reverse solid phase column, recovering a 40 to 50% v/v aqueous acetonitrile eluted fraction displaying sedative, sleep inducing or anxiolytic activity.

Steps (e, f, g) may also comprise *in vivo* and/or *in vitro* testing of the BDZ-bs binding and sedative, sleep and/or anxiolytic inducing activities of the various fractions from ether extract first portion and/or remaining aqueous portion.

Typically, the valeriana roots are *Valeriana wallichii* roots.

The active component obtained in step (f) may be hesperidin, and preferably the 2(S)-form of hesperidin, and the active component obtained in step (e) may be 6-methylapigenin.

Additionally, the valeriana roots used may be *Valeriana officinalis* wherein in step (f) linarin may be obtained as a novel active component. Linarin has never before been identified in *Valeriana officinalis*. The present invention therefore also relates to the identification of linarin in *Valeriana officinalis* and a method for isolating linarin from *Valeriana officinalis*.

In step (f) a silica-gel column may be used.

The novel fraction obtainable, preferably from the ether extract, by the above process, has been found to have particularly high levels of activity in relation to *in vitro* benzodiazepine binding site (BDZ-bs) binding activity, and *in vivo* anxiolysis. The novel fraction may comprise 6-methylapigenin. In particular, sedative, sleep and/or the anxiolytic activity has been found to be of a much higher order than that found with any other previously known valerian extracts. In more detail the active components of the present invention have been found to be capable of canceling the anxiety, inducing sleep and/or acting as a sedative in 30g body weight mice at a dosage of the order of 10 µg of active component (corresponding to a dosage of the order of 300 µg/kg body weight). This may be compared with required dosages of the order of at least 200 mg of conventional ethanolic extract per kg body weight or at least 1000 mg of whole root powder per kg body weight to obtain the same results. The active components of the present invention, alone or in combination are shown to possess comparable levels of greater activity than other conventional preparations such as those based on valepotriate mixtures, baldrinal, valerenic acid, and valeranone, whilst being substantially free or reduced in undesirable side effects such as those associated with some of these known preparations. The amounts of material recovered in the fractions comprising the active components of the present invention moreover represent a 500 fold purification relative to the dry root material and a 100 fold purification relative to a generally conventional 70 % v/v aqueous ethanol extract of the dry root material.

In another aspect, the method of the present invention provides an active sedative, sleep inducer and/or anxiolytic component comprising 6-methylapigenin. The 6-methylapigenin is present in the ethyl ether extract first portion. Additionally, another sedative flavonoid may be isolated and identified from the remaining aqueous portion.

The present invention also claims that the aqueous remaining portion after the petroleum ether and ethyl ether extraction may be used directly as a sedative dietary supplement. This aqueous remaining portion may comprise hesperidin and, in particular, S(-)hesperidin and linarin. To obtain the hesperidin amyl alcohol extraction may be used, followed by an evaporation technique to obtain a solid residue, and then by performing, for example, a silica-gel chromatographic technique the hesperidin may be obtained. To obtain the hesperidin and/or linarin, an amyl alcohol extraction may be used, followed by an evaporation step to obtain a solid residue, and then by performing, for example, a silica-gel chromatographic technique, the hesperidin and/or linarin may be obtained as separate peaks.

In a yet further aspect the present invention provides for the use of S(-)hesperidin, or 6-methylapigenin, or linarin, any combination thereof or with any other anxiolytic compound, including flavonoids such as disclosed in PCT/GB98/00906, PCT/GB94/01803, and PCT/GB96/02523 for the manufacture of a medicament for use in sedation, tranquilizing or sleep induction in an animal, such as mammals, including humans. When S(-) hesperidin and 6-methylapigenin are used in combination, the 6-methylapigenin is found to have a potentiating and/or synergistic effect. Linarin may also be used in combination with valerenic acid wherein the valerenic acid is found to have a potentiating and/or synergistic effect.

In a further aspect the present invention provides active components capable of inducing decreasing anxiety, inducing sedation and/or inducing sleep in mammals and obtainable from valerian roots by a process of the present invention, said components having an anxiolytic, sedation and/or sleep inducing activity corresponding to at least 10 times, preferably at least 20 times, advantageously at least 50 times, most preferably at least 100 times, most desirably at least 200 times, greater than that of whole root on weight for weight basis. In a yet another aspect the present invention provides active components capable of inducing sedation, anxiolysis and/or sleep in mammals and obtainable from valerian roots by a process of the present invention, said components having a sedative activity corresponding to at least 10 times, preferably at least 30 times, advantageously at least 50 times, greater than that of a 70 % v/v aqueous ethanol extract.

Preferably, the sedative, anxiolytic and/or sleep inducing dietary supplement as well as the single compounds of the invention are substantially free of at least one of the following compounds: valepotriates, baldrinals, and valerenic acid, and is desirably substantially free of all of these. Most preferably the active components of the invention have no more than 3 % w/w, advantageously not more than 1 % w/w, of these substances.

### DESCRIPTION OF THE INVENTION

It will be understood that various extraction techniques and procedures known in the art may be used for obtaining the novel active components of the present invention. Thus procedures such as soxhlet extraction, batch extraction, and continuous extraction may be employed with various non-polar and polar organic solvents and mixtures thereof with water and/or with each other. Suitable polar solvents that may be mentioned include methanol, ethanol, amyl alcohol, acetonitrile, diethyl ether, ethyl acetate and the like. Fractionation of crude extracts is generally carried out using chromatography, for example HPLC reverse phase, column chromatography using polar organic solvents such as those mentioned above and solid phases such as for example synthetic silica with C18 ligands (e.g. C18 reverse phase columns) available under the Vydac (Trade Name) (from the Separation Group Hesperia, CA, USA) and techniques such as gradient elution, isocratic separation, and stepwise elution. Chromatography on silica-gel columns eluted with methanol gradients in chloroform may also be employed.

It will be appreciated that there is a considerable number of different valeriana species (well over 100). Greater or lesser amounts of the active component of the present invention may be obtained from various different species including inter alia *Valeriana wallichii* D.C., *Valeriana officinalis* and *Valeriana edulis.*

The active components of the present invention are indicated as being useful in the sedation of mammals, and especially humans, for example suffering from sleep disorders such as insomnia. Thus in a further aspect the present invention provides a method of sedation of a mammal comprising the administration of a clinically useful amount of an active component of the present invention, a mixture of them or an analogue thereof, in a pharmaceutically useful form. The active components of the invention may be administered by any conventional route but generally are administered parenterally e.g. intravenously, or, most conveniently, orally. The active components of the invention are generally administered more or less immediately before sedation is required, sleep can typically be achieved within 30 minutes.

It will be appreciated that the amount of active component required to provide effective sedation will vary and ultimately is at the discretion of the medical or veterinary practitioner. The factors to be considered include the level or depth of sedation required, as well as the route of administration, the nature of the formulation, and the mammal's bodyweight, age and condition. Typically though a suitable dosage would be in the range of from 15 to 150 µg/kg bodyweight, preferably from 30 to 80 µg/kg bodyweight. Thus in the case of a 70 kg body weight human, a typical dosage would be of the order of from 1 mg to 10 mg of the active component, for example from 2.5 to 6 mg.

The present invention also provides in a further aspect the active components of the present invention for use in the preparation of sedatives.

Whilst it is in principle possible for the active components to be administered alone, it is preferable to present the active compounds in a pharmaceutical formulation. Formulations of the present invention, for medical use, comprise an active component of the present invention on a pharmaceutically acceptable carrier and optionally other therapeutic ingredients. The carrier(s) should be pharmaceutically acceptable in the sense of being compatible with the other ingredients of the formulation and not deleterious to the recipient thereof.

The present invention, therefore, further provides a pharmaceutical formulation of an active component of the present invention or a pharmaceutically acceptable salt or physiologically functional derivative or precursor thereof together with a pharmaceutically acceptable carrier therefor.

There is also provided a method for the preparation of a pharmaceutical formulation comprising bringing into association an active component of the present invention or a pharmaceutically acceptable salt or physiologically functional derivative thereof, and a pharmaceutically acceptable carrier thereof.

Formulations according to the present invention include those suitable for oral, topical (including pulmonary), rectal or parenteral (including intravenous) administration. Preferred formulations are those suitable for oral, or parenteral administration.

The formulations may conveniently be presented in unit dosage form and may be prepared by any of the methods well known in the art of pharmacy. All methods include the step of bringing the active compounds into association with carrier which constitutes one or more accessory ingredients. In general, the formulations are prepared by uniformly and intimately bringing the active compounds into association with a liquid carrier or a finely divided solid carrier or both and then, if necessary, shaping the product into desired formulations.

Formulations of the present invention suitable for oral administration may be presented as discrete units as capsules, cachets, tablets or lozenges, each containing a predetermined amount of the active compound; as a powder or granules; or a solution or suspension in an aqueous or nonaqueous liquid such as a syrup, an elixir, an emulsion or a draught. Other kinds of formulations such as teas or infusions may be also be used.

A tablet may be made by compression or moulding, optionally with one or more accessory ingredients. Compressed tablets may be prepared by compressing in a suitable machine the active compounds in a free-flowing form such as a powder or granules, optionally mixed with a binder, lubricant, inert diluent, surface active or dispersing agent. Moulded tablets may be made by moulding in a suitable machine a mixture of the powdered active compounds with any suitable carrier.

A syrup may be made adding the active compounds to a concentrated, aqueous solution of a sugar, for example sucrose, to which may also be added any accessory ingredients. Such accessory ingredient(s) may include flavorings, an agent to retard crystallization of the sugar or an agent to increase the solubility of any other ingredients, such as a polyhydric alcohol for example glycerol or sorbitol.

Formulations for rectal administration may be presented as a suppository with a conventional carrier such as cocoa butter.

Formulations suitable for parenteral administration conveniently comprise a sterile aqueous preparation of the active compounds, which is preferably isotonic with the blood of the recipient.

Useful formulations also comprise concentrated solutions or solids containing an active component of the present invention, which upon dilution with an appropriate solvent give a solution for parenteral administration as above.

For pulmonary administration, the combination is suitably inhaled from a nebulizer, from a pressurized metered dose inhaler or as a dry powder from a dry powder inhaler or from a dry powder inhaler utilizing gelatin, plastic or other capsules, cartridges or blister packs. A diluent or carrier, generally non-toxic and chemically inert to the medicament e.g. lactose, dextrin, mannitol or glucose or any additives can be added to the powdered medicament. The agglomerated, free-flowing micronized mixture may be filled into a dry powder inhaler. When a capsule system is used, it is desirable to include a filler in the mixture.

The micronized mixture may be suspended or dissolved in a liquid propellant mixture which is kept in a container that is scaled with a metering valve and fitted into a plastic actuator. The propellants used may be chlorofluorocarbons of different chemical formulae.

In addition to the aforementioned ingredients, formulations of this invention may further include one or more accessory ingredient (s) selected from diluents, buffers, flavoring agents, binders, surfactants, thickeners, lubricants, preservatives (including antioxidants) and the like.

As noted above, it is a particular problem of previously known valerian preparations, that the effective activity thereof has been unknown and/or highly variable. In yet another aspect the present invention now provides a method of preparing a valerian-based sedative preparation with a standardized activity comprising the steps of: preparing a valerian extract containing a pharmacologically effective amount of an active component of the present invention; assaying the BDZ-bs binding activity of said extract; and making a preparation containing an amount of said extract providing a total amount of BDZ-bs binding activity alone or together with an amount of said hesperidin, estimated by HPLC, corresponding to a desired sedation, anxiolysis or/and sleep inducing activities. It should be considered, in these preparations, the potentiation that occurs when 6-methylapigenin is administered simultaneously with hesperidin. The potentiation being a novel fact established in the present invention.

Further preferred features and advantages of the invention will appear from the following illustrative Examples and Figures wherein:
Figures 1a and 1b are HPLC chromatograms of fractionated *Valeriana wallichii* extracts: 1a: Ethyl ether extract (see Figure 2); 1b: Aqueous remaining solution (see Figure 2);
Figure 2 is a flow-sheet of a *Valeriana wallichii* fractionation scheme on a semi-preparative scale;
Figure 3 is a graph indicating the inhibition of tritiated flunitrazepam ([³H] FNZ) binding to the BDZ-bs by *Valeriana wallichii* fractions obtained in the C18 HPLC fractionation (see Figure 2);
Figure 4 is a semi-preparative HPLC chromatogram of a dry sample of the active fractions obtained in the silica-gel chromatography of fraction H (see Example 3) eluted with a gradient of acetonitrile (ACN);
Figure 5 is the molecular structure of 6-methylapigenin;
Figure 6 is a Scatchard graph of saturation curves of [³H] FNZ binding in both the presence and absence of 6-methylapigenin;
Figure 7 is the molecular structure of hesperidin;
Figure 8 is the HPLC chromatograms of valeriana and standard hesperidins;
Figure 9 is the mechanism of the reversible transformation of hesperidin into chalcone; and
Figure 10 is the CD spectra of valeriana hesperidin and of citrus hesperidin (Sigma).

### Example 1 - Analytical Scale Fractionation of Valeriana wallichii Extracts

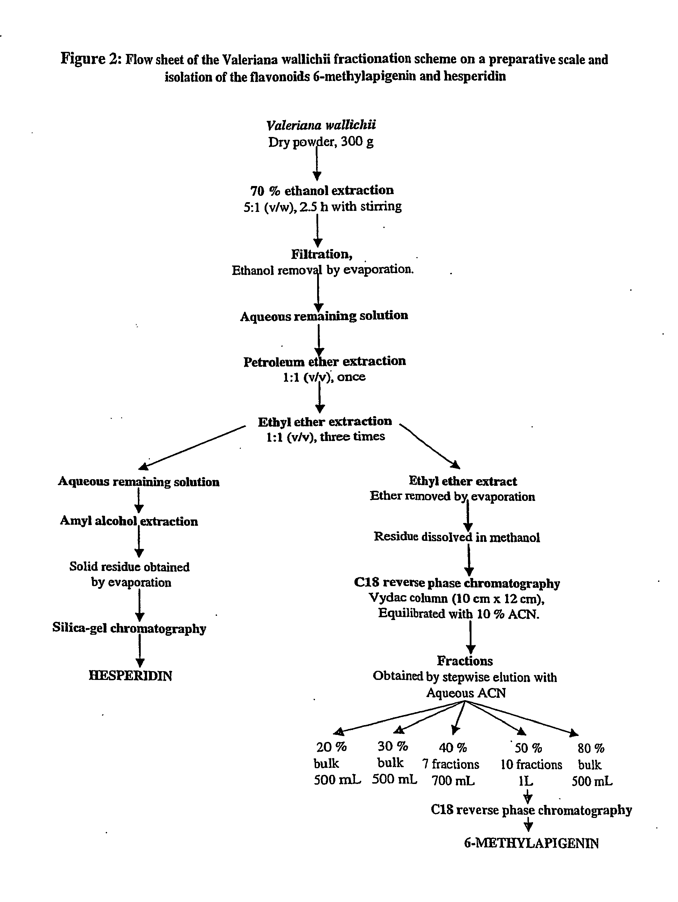

Dry roots and rhizomes powder from *Valeriana wallichii* was extracted and fractionated as described in Figure 2. The ethanol solution was concentrated in vacuum to 1/3 of its original volume and the remaining aqueous solution was extracted with 1 volume of petroleum ether, that was discarded. The aqueous phase was extracted 3 times with an equal volume of ethyl ether. The ether was evaporated and the dry residue dissolved in methanol. A sample of the aqueous remaining solution was also evaporated under vacuum and the residue dissolved in aqueous methanol.

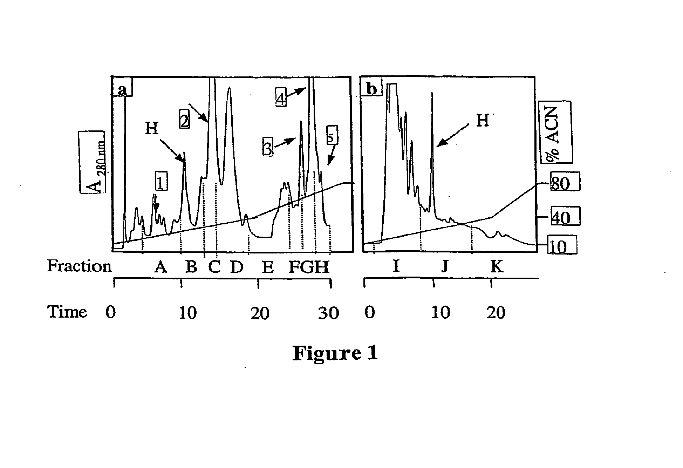

The resulting ethyl ether extract from the 70 % aqueous ethanol extract, and the extract from the aqueous remaining solution, were then subjected to HPLC analysis using the following procedure: the column used was a C18 Vydac (Trade Name) analytical column. Each extract was properly injected into the column and eluted using an aqueous ACN gradient from 10 % to 80 % v/v as indicated in Figure 1. Monitoring of the effluent was at 280 nm. Panels a and b correspond to the ethyl ether extract and aqueous remaining solution, respectively (see Figure 2).

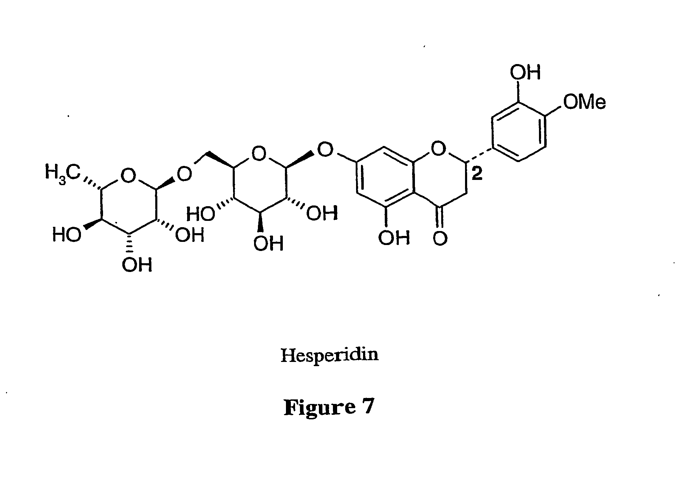

The results of the HPLC analyses of the above-mentioned extracts are shown in Figure 1. Fractions A to K were recovered for use in biological testing in Example 2. The numbered arrows in the HPLC diagrams of Figure 1 indicate the retention times of the following *Valeriana wallichii* components: a, 1 and 2: baldrinals; a, 3: acevaltrate; a, 4:didrovaltrate and a, 5: valtrate. In a and b, H indicates the eluted peak with the retention time of the novel component hesperidin (see Figure 7) described in this invention. Valerenic acid (which is absent in *Valeriana wallichii*, but present in *Valeriana officinalis*), has a retention time intermediate between those of a, 4 and a, 5.

In summary: the whole ethanolic extract of valerian roots has been effectively fractionated so as to provide discrete fractions containing baldrinals which arise as decomposition products of the valepotriates, valepotriates, valerenic acid (if present), hesperidin, 6-methylapigenin, and other unknown or unidentified components.

### Example 2 - Investigation of the biochemical and pharmacological properties of Valeriana wallichii extract fractions obtained in the experiment shown in Figure 1

*In vitro* binding assays were carried out with various receptors as well as *in vivo* tests for sedative activity on the various fractions A-K recovered in Example 1 using the known procedures described in detail in the literature for the following receptors: BDZ-bs (benzodiazepine binding site in GABA_{A} brain receptor) (1); 5-hydroxytriptamine-type 1A (5HT1_{A}) (2); 5HT2 (3); AMPA glutamatergic (4) ; adenosine 1 (5) . *In vivo* sedation activity was measured by the Hole-board test (6).

### RESULTS

The results of the tests are shown in Table 1 in which relatively low and high activity are indicated as + and +++, respectively and zero activity as -.

It was surprisingly found that presumably high affinity ligands for the BDZ-bs were present in a single discrete fraction not containing valepotriates, baldrinals or valerenic acid. It should be remembered that these compounds were traditionally held responsible of the majority of the *in vivo* actions of valerian. We also found in this fraction some proportion of ligands recognizing the 5HT1_{A} and adenosine receptors. Without in any way wishing to restrict the scope of the present invention it is believed that the sedative effects detected *in vivo* with fractions B, C, D, G, H and J, can be partially explained on the basis of fractions B and J containing hesperidin (see Example 9), and/or 6-methylapigenin (see Example 7), this last compound being a novel ligand for the BDZ-bs; fraction C containing baldrinals and fraction G and H containing valepotriates.

The aqueous remaining solution also has sedative properties when it is assayed unfractionated, but as shown in Table 1 only fraction J is active in the sedation test. However, these fractions (I, J and K) appear to interact synergistically in the unfractionated aqueous remaining solution to produce increased sedative and sleep inductive effects (see Table 6).

**Table 1 - Results of Receptor Binding Assays and Sedation Activity Testing**

| Receptors and sedative activity | Fractions / binding level | | | | | | | | | | |
|---|---|---|---|---|---|---|---|---|---|---|---|
| | A | B | C | D | E | F | G | H | I | J | K |
| **BDZ** | - | - | - | +++ | - | - | - | - | - | - | - |
| **5 HT_{1A}** | + | - | + | + | + | + | - | + | - | - | - |
| **5 HT₂** | - | - | - | - | - | - | - | - | - | - | - |
| **AMPA glutamatergic** | - | - | - | - | - | - | - | - | - | - | - |
| **Adenosine₁** | - | - | - | + | - | - | - | - | - | - | - |
| **Sedative activity** | - | ++ | + | +++ | - | - | + | + | - | + | - |

**Table 2 - Correspondences between Valeriana wallichii fractions in Figures 1 and 2**

| Figure 1 HPLC fractions | Figure 2 % ACN fractions |
|---|---|
| A | 20 |
| B | 30 |
| C | 40 |
| D | 50 |
| E, F, G, H | 80 |
| I, J, K | Aqueous remaining solution |

### Example 3 - Preparative scale fractionation of Valeriana wallichii extracts

Dry powdered *Valeriana wallichii* roots (300 g) were extracted and fractionated using the flow-sheet shown in Figure 2. The close correspondence between the fractions obtained with those shown in Figure 1a (Table 2) was established by HPLC analysis of each fraction using the same procedures described in Example 1. In the same way the aqueous remaining solution was found to correspond with the fractions in Figure 1b.

**Table 3 - Volume and dry residue weight of the fractions of Valeriana wallichii obtained by application of the flow-sheet scheme shown in Figure 2**

| **Fraction** | **Description** | **Volume (mL)** | **Dry residue weight** | |
|---|---|---|---|---|
| | | | Total (mg) | mg/g valeriana* |
| A | 70 % Ethanol extract | 2000 | 59000 | 197 |
| B | Aqueous remaining solution | 600 | 49000 | 163 |
| C | Ethyl ether extract | --- | --- | --- |
| | C18 HPLC eluate fractions: | | | |
| D | 20 % ACN fractions 1-5, bulk | 500 | 390 | 1.3 |
| E | 30 % ACN fractions 6-10, bulk | 500 | 175 | 0.58 |
| F | 40% ACN separate 100 ml fractions. Pool: 11-17 | 700 | 1098 | 3.66 |
| | 50 % ACN separate 100 mL fractions. Pools: | | | |
| G | 18-22 | 500 | 425 | 1.42 |
| H | 23-27 | 500 | 600 | 2.00 |
| I | 80 % ACN fraction 28-32, bulk | 500 | --- | --- |

| | | | | |
|---|---|---|---|---|
| * Dry root powder | | | | |

Further details of the preparative fractionation scheme of Figure 2 are shown in Table 3 herein above which includes the amounts of material recovered in the various extracts and fractions.

### Example 4 - BDZ-bs binding activity of Valeriana wallichii fractions derived from the ethyl ether extraction

The presence of BDZ-bs ligands in the fractions obtained at the end of the preparative fractionation scheme described in Figure 2 was checked by the BDZ-bs binding assay as described in Example 2.

### RESULTS

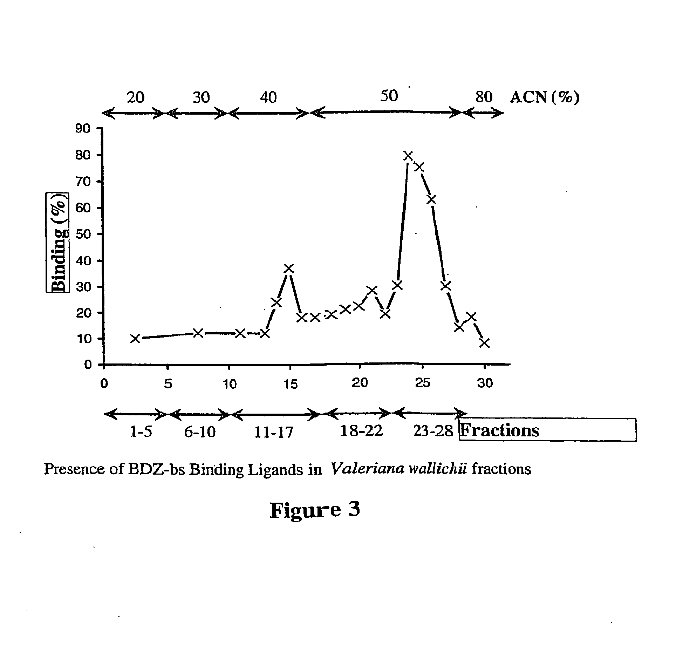

No BDZ-bs ligands were found in the eluates obtained with 20 %, 30 % and 80 % ACN, but they were present in those obtained with 40 % and 50 % ACN. The BDZ-bs ligands distribution obtained is shown in Figure 3 in which the percentage of binding inhibition of [³H]-FNZ to rat cerebral cortical membranes (averages of duplicated measurements), is shown for 0.05 mL aliquots of the indicated fractions. (Note: Fractions 1-5, 6-10 and 28-32 were collected in bulk; 40 % and 50 % eluates were collected as separate 100 mL fractions. The arrows inside the graph indicate the pools made). These findings confirm the existence of ligands for the BDZ-bs already shown in Figure 1 with the additional information of the finding of their heterogeneity.

The above findings, together with those of Example 2, show that the preparative method provides a novel fraction containing components with substantially all of the BDZ-bs binding activity in *Valeriana wallichii* and which is substantially free from other known components such as valepotriates, baldrinals and valerenic acid. Fractions 23-27 (Figure 3), which contain the major proportion of the BDZ-bs ligands, has a total dry weight residue of 600 mg and represents a 500 fold purification from the starting dry powdered root (300 g) (Table 3).

### Example 5 - Sedative activity of Valeriana wallichii fractions derived from the ethyl ether extraction

In all *in vivo* tests male Swiss mice of 28-30 g were used. The animals were injected i.p. with vehicle, or a solution of the drug, or fraction, 20 min before the assay. The assays were performed in mice i.p. injected with an amount of each fraction equivalent to that obtained from 1 g of dry *Valeriana wallichii* powder (see Table 3), 20 min before the test. In the case of the remaining aqueous solution (see Figure 2) it was injected at the maximum tolerated dose which was equivalent to that obtained from only 12 mg of dry *Valeriana wallichii* powder. This low dosage explains the absence of activity recorded in Table 4.

The following tests were used: spontaneous ambulatory activity counts in an Opto-Varimex apparatus that optically detects and measures locomotor activity; the Hole-board, a well-known test to assay for the sedative behavior (6); and the horizontal wire test to detect myorelaxation as described in detail in the literature (7).

### RESULTS

The results of the tests are shown in Tables 4 and 5 and are expressed as percentage of decrements relative to vehicle (VEH) values; n: number of mice.

**Table 4 - Effect in different behavioral test of fractions of Valeriana wallichii**

| **Valerian Fraction# (Table 3)** | **n** | **Locomotor Activity** | **Hole-board test** | | | **Wire** |
|---|---|---|---|---|---|---|
| | | | **N° of Head-dips** | **Time head dipping** | **N° of rearings** | |
| VEH | 20 | 100 | 100 | 100 | 100 | 0 |
| Aqueous | 7 | -21 | -18 | -25 | -28 | 0 |
| ACN 20 % (bulk) | 8 | -50 | -34 | -42 | **-49*** | 0 |
| ACN 30 % (bulk) | 8 | 0 | -35 | **-49*** | -19 | 0 |
| ACN 40 % (11-17) | 9 | 0 | -29 | -36 | -14 | 0 |
| ACN 50 % (18-22) | 8 | -15 | **-44*** | -44 | **-60**** | 0 |
| ACN 50 % (23-27) | 8 | -**76##** | **-85**** | **-87*** | **-81**** | 22 |

| | | | | | | |
|---|---|---|---|---|---|---|
| # The doses injected were equivalent to 1 g of dry powder of valeriana per mouse. ## p<0.01, Dunn's multiple comparison test (MCT). *p<0.05, **p<0.01, Dunnett's MCT. | | | | | | |

Table 4 shows the depressor effects obtained with the fractions described in Figures 2, 3 and Table 3. The most active fraction is ACN 50% (23-27) as was expected from the results in Figure 3, which indicates that this fraction contains the highest proportion of BDZ-bs ligands together with hesperidin (see Example 12). The depressor activity found with ACN 50% (18-22) can also be explained by the presence in this fraction of BDZ-bs ligands (Figure 3). However, the same occurrence of BDZ-bs ligands in ACN 40% (11-17) is apparently not expressed *in vivo* by a depressor activity. The moderate *in vivo* activity of fractions ACN 20% and 30% can be explained by the presumable presence of baldrinals in these fractions (see Figure 1a).

**Table 5 - Relationship between doses and depressor effects in mice of Valeriana wallichii fraction ACN 50 % (23-27).**

| **Dose of ACN 50 % (23-27) (g/mouse)*** | **n** | **Locomotor Activity** | **Hole-board test** | | | **Wire** |
|---|---|---|---|---|---|---|
| | | | **N° of Head-dips** | **Time head-dipping** | **N*°* of rearings** | |
| VEH | 18 | 100 | 100 | 100 | 100 | 0 |
| 0.01 | 6 | 0 | -30 | -30 | -24 | 0 |
| 0.05 | 6 | -13 | **-41*** | -29 | -29 | 0 |
| 0.1 | 7 | **-46** | **-55**** | **-57**** | -13 | 0 |
| 0.5 | 8 | **-75#** | **-63**** | **-65**** | **-64**** | 22 |
| 1 | 8 | **-78##** | **-84**** | **-85**** | **-81**** | 62+ |
| 5 | 8 | **-87##** | **-86**** | **-87**** | **-98**** | **38+** |

| | | | | | | |
|---|---|---|---|---|---|---|
| Results expressed as in Table 4. *Equivalent to dry g of powder of Valeriana per mouse. # p<0.05, ## p<0.01, Dunn's MCT. * p<0.05, ** p<0.01, Dunnett's MCT. + p<0.05, Chi Square test. | | | | | | |

Table 5 shows that significant sedative effects occur with doses of fraction ACN 50% (23-27) as low as that equivalent to 0.1 g of dry *V. wallichii* powder per mouse. The locomotor activity and the muscle tone (wire test), require higher doses to be effective.

### Example 6 - Effect of Valeriana wallichii ethyl extract fraction H ACN 50%, (23-27) (Table 3) and aqueous remaining solution, (fraction B Table 3) on sleeping time in mice

The effect of the active components in fraction H of Table 3 (ACN 50%, 23-27) and that of the aqueous remaining solution (Table 3) were tested in mice by i.p. administration. The doses were 2 mg per mouse for fraction H and 5 mg per mouse for the aqueous remaining solution fraction B. A small amount of conventional anesthetic (30 mg/kg of thiopental sodium - Biochemie, GmbH, Kundl, Austria) was used to induce a slight sleeping condition in the mice which were monitored to detect any possible enhancement of the sleeping condition by the previous injection of the novel compounds.

The results shown in Table 6 indicate that the average sleeping time using 2 mg of the active component fraction H was 14 minutes as compared with an average sleeping time of 0.4 minutes for the controls. The aqueous remaining solution (Fraction B) was also active, increasing the sleeping time to 16 minutes.

**Table 6 - Effectiveness of fraction B (Table 3) and of fraction H (Table 3) on sleeping time of mice**

| **Sample** | **Dose (mg/mouse)** | **n** | **Sleeping time (min) median values** |
|---|---|---|---|
| VEH | -- | 11 | 0.4 |
| Fraction B | 5* | 14 | 16 |
| Fraction H | 2^{#} | 9 | 14 |

| | | | |
|---|---|---|---|
| * Dry residue weight equivalent to 1 g of Valeriana powder per kg. # Dry residue weight equivalent to 30 g of Valeriana powder per kg. | | | |

In Examples 7 and 8, that follow, we describe the isolation and identification of a novel ligand for the BDZ-bs from Fraction H and of another novel sedative compound from Fraction B.

### Example 7 - Preparative-scale fractionation of Valeriana wallichii extracts at the 40 kg level. Isolation of a pure ligand for the BDZ-bs from the ethyl ether fraction (Example 3 and Figure 2).

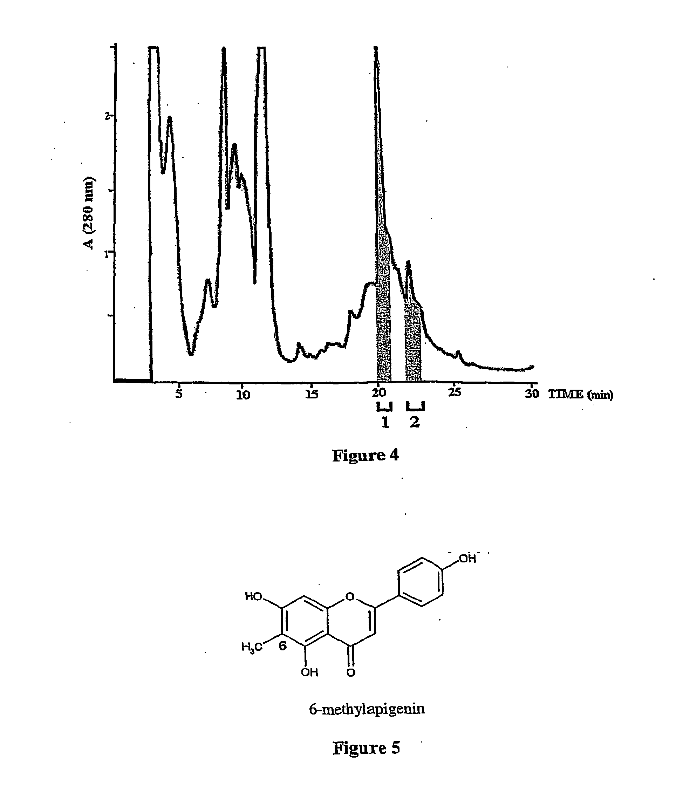

When the method described to purify 300 g of *Valeriana wallichii* (Example 3) was applied to 40 kg of starting material it was possible to further purify Fraction H (Table 3) by two chromatographic steps as follows:
a) Silica-gel chromatography: Fraction H obtained in a preparative C18 chromatography (see Example 3) was purified in a 4.6 cm per 20 cm column of silica gel prepared from a suspension of silica H (Sigma) in chloroform. A dry sample of Fraction H corresponding to 5 kg of starting Valeriana powder, dissolved in chloroform, was added to the column, which was eluted stepwise with 280 mL/per step, of chloroform with 0, 1, 4, 6, 8 and 10% methanol, respectively. The fractions active in the BDZ-bs binding assay were pooled and evaporated to dryness.
b) HPLC semi-preparative: This step was performed in a C18 semi-preparative column (1 cm x 25 cm; 201 HS1010, Vydac (Trade Name) connected to a high flow ISCO HPLC chromatograph. A dry sample, obtained from the silica-gel step, was chromatographed with a linear gradient of ACN rising in 20 min from 20 % to 50 % in water followed by an isocratic step of 10 min. The flow rate was 5 mL/min and the eluate was monitored by UV absorption at 280 nm and by its activity in the BDZ-bs binding assay. Two active fractions were detected (Figure 4, fractions 1 and 2). Fraction 1 yielded a yellow precipitate, which upon recrystallization from ethanol/water, gave pure 6-methylapigenin (approximately 10 mg) (see Figure 5). Fraction 2 yielded less than 500 µg.

### Identification of isolated compounds:

6-methylapigenin: yellow crystals from ethanol/water. UV λₘₐₓ 350 (sh), 318, 271, 211 nm. ¹H NMR δ_{H} 7.91 (2H, d, J= 8.9 Hz, H-2' and H-6'), 6.91 (2H, d, J= 8.9 Hz, H-3' and H-5'), 6.77 (1H, s, H-3), 6.53 (1H, s, H-8), 1.97 (3H, s, Me). ¹³C NMR δ_{c} 181.7 (C-4), 164.3 (C-7), 163.8 (C-2), 161.1 (C-4'), 160.0 (C-5), 158.5 (C-9), 128.4 (C-2' and C-6'), 121.3 (C-1'), 115.9 (C-3' and C-5'), 108.5 (C-6), 106.8 (C-10), 102.8 (C-3), 93.0 (C-8). EIMS *m*/*z* 284, 267, 255, 239, 167, 166, 165, 138, 121.

Compound 2: could not be identified by ¹H NMR due to its low yield. Its EIMS *m*/*z* M⁺ was 368.

Hence, a BDZ-bs binding bioassay-directed fractionation of the ethyl ether fraction derived from *Valeriana wallichii* ethanol extracts permitted the isolation of two novel ligands, 1 and 2, with Ki values of 0.50 ± 0.17 µM (n= 5) and 1.3 ± 0.6 µM (n= 3), respectively, for the BDZ-bs.

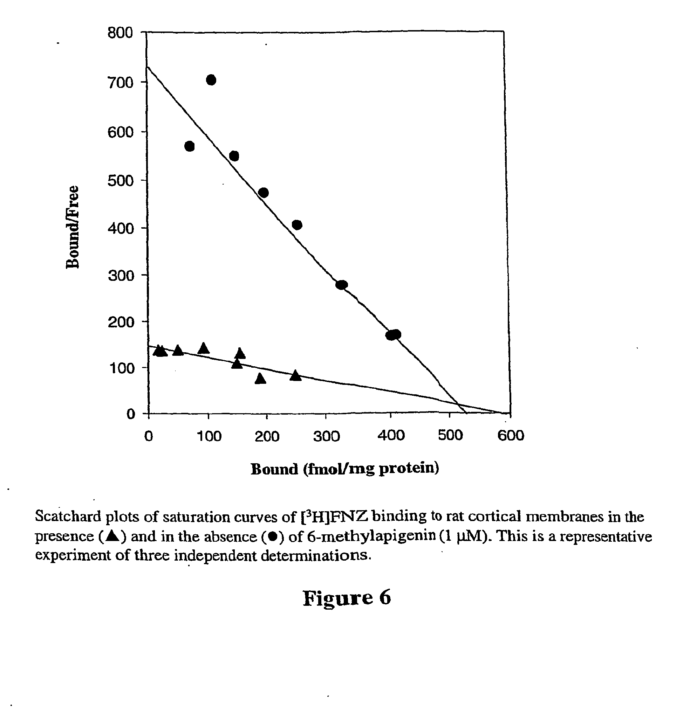

Scatchard analysis of saturation curves for compound 1 revealed a competitive interaction showing a decline in the apparent affinity without changes in the maximal number of sites (Bmax) (Figure 6). Displacements curves of [³H]-FNZ binding to cortical brain membranes, in the presence or absence of 100 µM GABA (GABA-shift, 8), showed that compound 1 behaves as an agonist (GABA-shift of compound 1= 1.6-2.0, n= 3); experiments run in parallel with diazepam gave a GABA-shift of 2.00 ± 0.10 (SEM, n= 3).

This is the first record in the literature of 6-methylapigenin in a Valeriana species and also of its ability to act as a competitive ligand for the BDZ-bs.

### Example 8 - Pharmacological properties of 6-methylapigenin

6-methylapigenin up to 1 mg/kg does not affect the sleeping time of mice receiving a sub-hypnotic dose of thiopental (see Example 6 for technical details) (Table 7). Also, up to 10 mg/kg 6-methylapigenin does not have sedative effects in mice (Table 8).

**Table 7 - Effect of 6-methylapigenin on sodium thiopental-induced sleeping time**

| **Sample** | **Dose (mg/kg of mice)** | **n** | **Sleeping time (min) median values** |
|---|---|---|---|
| VEH | -- | 15 | 0 |
| 6-methylapigenin | 1 | 8 | 0 |

Median (interquartile range) of sleeping time of mice given a sodium thiopental i.p. administration (30 mg/kg) 20 min after an i.p. injection of vehicle or 6-methylapigenin. The sleeping time was measured as the time spent between disappearance and reappearance of righting reflex.

**Table 8 - Effect in different behavioral tests of 6-methylapigenin (sedation tests)**

| **Sample** | **n** | **Total Locomotor Activity** | **Ambulatory locomotor activity** | **Hole-board test** | | | **Wire** |
|---|---|---|---|---|---|---|---|
| | | | | **N° of Head-dips** | **Time head dipping** | **N° of rearings** | |
| VEH | 5 | 634±12 | 446±14 | 7±1 | 12±3 | 26±4 | 5/5 |
| 6-methylapige nin (10 mg/kg of mice) | 5 | 552±55 | 395±43 | 7±0.5 | 10±1 | 28±3 | 5/5 |

Mean ±SEM of locomotor activity registered during 5 min session in an Opto-Varimex apparatus and performance of mice in the holeboard test. Wire test was performed after two trials and was registered the number of animals grasped the wire for 3 seconds.
Vehicle or 6-methylapigenin were ip administered 20 min before tests.
No significant differences were observed using the Student's t test at p>0.05.

**Table 9 - Anxiolytic effect of 6-methylapigenin measured in the plus-maze test**

| **Sample** | **n** | **Locomotor Total activity** | **arm entries** | **% open arm entries** | **% time spent in open arms** |
|---|---|---|---|---|---|
| Vehicle | 13 | 405±32 | 18±2 | 35±3 | 29±4 |
| 6-methylapigenin 0.3 mg/kg | 14 | 410±36 | 17±2 | 48±4 | 42±4 |
| 6-methylapigenin 1 mg/kg | 12 | 463±64 | 14±2 | 60±6** | 58±5** |

| | | | | | |
|---|---|---|---|---|---|
| Means ±SEM of ambulatory locomotor activity registered during 5 min session in an Opto-Varimex apparatus and performance of mice in the plus-maze test. Vehicle or drug were ip administered 20 min before tests. **No significant differences were observed using the Student's t test at p<0.01 different from vehicle, Dunnet's Multiple Comparison test after ANOVA. | | | | | |

6-Methylapigenin, tested in the plus-maze assay, proved to have clear anxiolytic properties at the dose of 1 mg/kg in mice.

### Example 9 - Preparative-scale fractionation of Valeriana wallichii extracts at the 40 kg level. Isolation of a novel sedative and sleep-inducing compound from the aqueous remaining solution (Figure 2).

The aqueous remaining solution (Figure 2) is effective in mice in the sleep inducing test (see Table 6) which suggests the presence of active components, presumably glycosidic and hence, extractable with amyl alcohol (9) (Figure 2). The solid residue from this extract, corresponding to 1 kg of starting Valeriana powder, dissolved in chloroform was added to a 4.5 cm x 20 cm column of silica gel prepared from a suspension of silica H (Sigma) in chloroform which was eluted with 20% methanol in chloroform. The fractions showing the presence of compounds active in the sleep inducing assay were pooled. After partial evaporation of the solvent the pool deposits a whitish precipitate that was collected by filtration and purified by recrystallization from warm ethanol. Yield: 0.5 mg per g of Valeriana powder, which means a concentration in this material of 5 mg/g, accounting for the estimated losses in the process.

### Identification of the isolated compound:

Hesperidin: Hesperetin 7-rhamnoglucoside; 4H-1-Benzopyran-4-one, 7-[[6-O-(6-deoxy-alpha-L-mannopyranosyl)-beta-D-glucopyranosyl]oxy]-2,3-dihydro-5-hydroxy-2-(3-hydroxy-4-methoxyphenyl)-, (S)- (Figure 7): white sphero crystals from ethanol. UV λₘₐₓ (methanol) 331.5, 283.5, 210 nm. ¹H NMR δ_{H} 12.00 (1H, s, OH-5), 9.04 (1H, s, OH-3'), 6.90 (3H, dd, J= 7.00 and 14.00 Hz, H-2', H-5'and H-6'), 6.11 (2H, d, J= 6 Hz, H-6 and H-8), 5.50 (1H, dd, J= 3 and 12 Hz, H-2), 4.96 (1H, d, J= 6 Hz, H-1 glucosyl), 4.40 (1H, d, J= 6 Hz, H-1 ramnosyl), 3.76 (3H, s, CH₃-4'), 3.10-3.28 (m, H-sugars and H-3 trans), 2.80 (1H, d, J= 3 Hz, H-3 cis), 1.06 (3H, s, CH₃ rhamnosyl). ¹³C NMR δ_{c} 197.07 (C-4), 165.13 (C-7), 163.04 (C-5), 162.50 (C-9), 147.95 (C-3'), 146.44 (C-4'), 130.87 (C-6'), 128.54 (C-1'), 117.97 (C-5'), 114.14 (C-2'), 111.97 (C-10), 100.62 (C-1 glucose), 99.38 (C-1 rhamnose), 95.52 (C-8), 78.37-68.32 (C-glucose and rhamnose), 66.02 (C-6 glucose), 55.66 (OCH₃-4'), 17.85 (C-6 rhamnose).

All the spectra were identical to those of authentic hesperidin (Sigma) obtained under the same conditions.

### HPLC identification of hesperidin

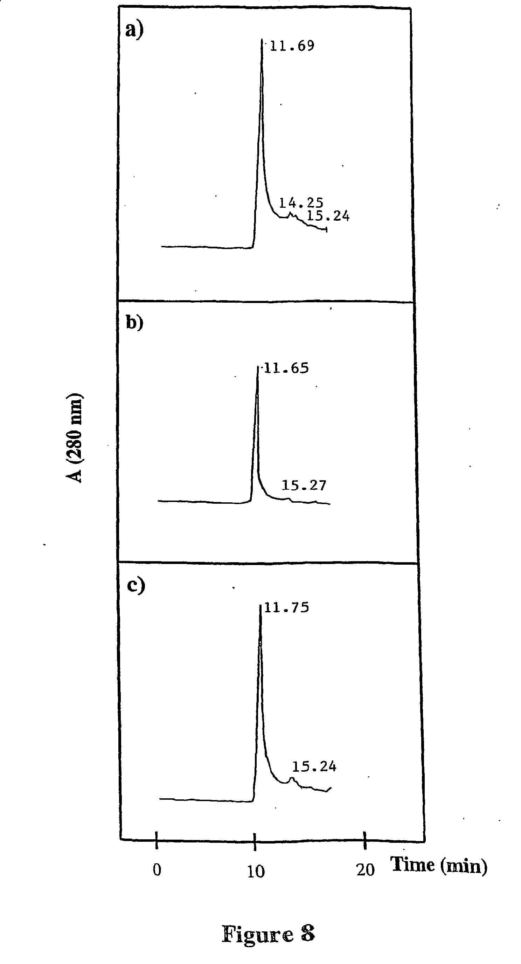

Three chromatographies were performed with the results shown in Figure 8. The HPLCs were done in an analytical C18 Vydac (Trade Name) column (1 cm x 25 cm; 201 HS1010, The Separation Group, Hesperia, CA, USA) eluted with a ACN-water gradient from 10 to 40 % ACN, in 20 min, as indicated, and scanned at 280 nm. Panel a is the chromatographic pattern obtained with 50 µg of the Valeriana compound; panel b, is a similar pattern obtained with 50 µg of authentic hesperidin (Sigma) and Panel c shows the diagram obtained in the co-chromatography of both samples. The retention time of hesperidin in this HPLC system is 11.7 min (cf. results shown in Figure 1).

### Identification of the sugars and of the aglycone in hesperidin

Samples of Valeriana hesperidin, or authentic hesperidin (Sigma), were hydrolyzed with boiling 1 M HC1, for 1 hour. The aglycones were extracted from the hydrolysates with ethyl ether. The ether extract residues were submitted to ¹H NMR analysis. Both spectra were identical:

Valeriana hesperidin or authentic hesperidin aglycon, hesperetin: ¹H NMR δ_{H} 12.11 (1H, s, OH-5) , 10.75 (1H, s, OH-7), 9.05 (1H, s, OH-3'), 6.90 (3H, m, H-2', H-5'and H-6'), 5.87 (2H, dd, J= 2 and 4.7 Hz, H-8 and H-6), 5.42 (1H, dd, J= 3 and 12.3 Hz, H-2), 3.76 (3H, s, CH₃-4'), 3.17 (1H, m, J= 4.7 and 12.3 Hz, H-3 trans), 2.73 (1H, d, J= 3 Hz, H-3 cis).

Sugars: Samples from the hydrolysated aqueous phases remaining after the ethyl ether extraction were submitted to TLC on silica gel on polyester sheets, with 254 nm fluorescent indicator (Sigma) developed with butanol/acetic acid/ethyl ether/water (9:6:3:1 v/v) as the solvent and stained using a general reactive for sugars (aniline/diphenylamine/acetone/phosphoric acid). The TLCs were performed alongside with authentic sugar standards. In both cases the only sugars detected were glucose and rhamnose.

### Optical activity of hesperidin

Stereochemical changes arise in natural hesperidin with variations in the pH.

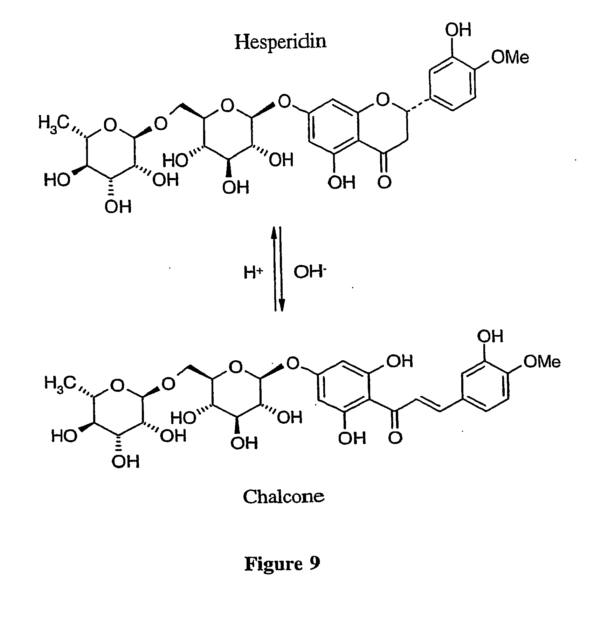

The disaccharide rhamnose-glucose, or rutinose, is glycosidically attached to C7 in hesperetin to give rise to hesperidin. Rutinose has optical activity due to several asymmetric carbons (Figure 7) and its contribution to the optical activity of hesperidin remains unchanged when the glycoside is submitted to mild treatments with alkali or acid. However, the bond between the asymmetric C2 and the pirone O is split by mild alkali (10) giving rise to chalcone (Figure 9) where the C2 asymmetry is lost.

The reversal of this reaction by acidification, originates a racemic form of hesperidin at C2. This racemate, however, is optically active due to the presence of the rutinose moiety in the molecule.

The Index Merck records for the specific optical rotatory power of 2S-hesperidin: [α]²⁰_{D}= -76° (c= 2 in pyridine).

Our own results with hesperidin obtained from *Valeriana wallichii* and with authentic hesperidin (Sigma), recrystallized from acetic acid, are:
hesperidin (*Valeriana wallichii) :* [α]^{t}_{D}= -84.5° (c= 0.02 in pyridine); and
hesperidin (Sigma) : [α]^{t}_{D}= -64.3° (c= 0.02 in pyridine).

The low rotatory power of the authentic hesperidin (Sigma) can be explained by its extraction from Citrus peels using alkaline solutions, later acidified (10), which brings about its partial racemization. We assume that the valeriana hesperidin obtained by us is the pure 2S(-) natural form. The difference in absolute values of [α] in Index Mark are probably due to the different concentrations used.

### Circular Dichroism (CD) Studies:

It is clear from the previous considerations that optical rotatory measurements are not suitable to ascertain the proportions of isomers in a particular sample of hesperidin.

CD measurements, in contrast, are appropriate for this purpose since they detect the contribution of each absorption band of the molecule and the sugar moiety, rutinose, does not interfere being devoid of absorptive bands in the UV.

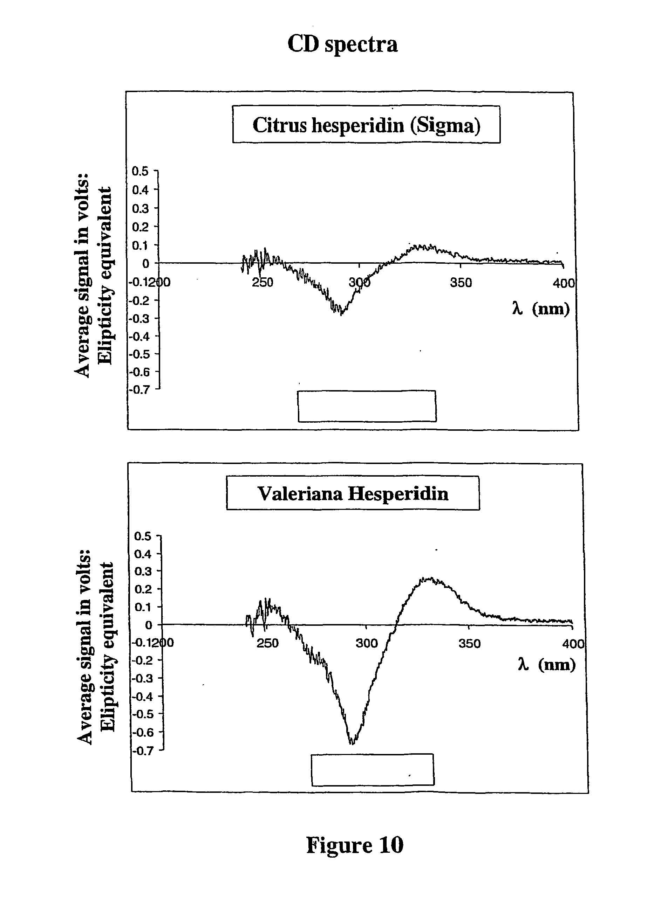

Figure 10 shows the CD spectra of recrystallized authentic hesperidin (Sigma) and of valeriana hesperidin, isolated in the present invention. The spectra were recorded in a Jasco-20 spectro-polarimeter, between 250 and 400 nm which includes the typical UV bands of hesperidin at 286, 289 and 330 nm.

Using the elipticity recorded at 330 nm for both hesperidins it can be calculated that the Citrus hesperidin is a mixture of 25 % free natural and 75 % racemic forms. Hence citrus hesperidin has only approximately 63 % of pharmacologically available S active form.

### Example 10 - Valeriana hesperidin and Citrus hesperidin (Sigma): comparative pharmacological assays

### Effect on sleeping time in mice

The assay was carried out as described in Example 6. The results are shown in Table 10 from which it may be estimated that the activity of the citrus hesperidin (Sigma) is approximately 60 % of the activity displayed by the natural isomer from valeriana. This in vivo results are in line with the analysis of the samples performed by CD.

Optical Rotatory and CD measurements indicate that the valeriana hesperidin is the 2S (-) isomer, while the citrus hesperidin (Sigma) contains only 63 % of the 2S (-) isomer.

**Table 10 - Effectiveness of Valeriana and Citrus (Sigma) hesperidins on sleeping time of mice**

| **Sample** | **Dose (mg/kg of mice)** | **n** | **Sleeping time Median (s)** |
|---|---|---|---|
| Vehicle | - | 21 | 0 |
| Valeriana hesperidin | 1 | 6 | 35 |
| | 2 | 15 | 197* |
| | 4 | 14 | 1290** |
| | 9 | 6 | 1800** |
| Citrus hesperidin | 4 | 7 | 660* |
| | 9 | 6 | 1440** |

| | | | |
|---|---|---|---|
| Median (interquartile range) of sleeping time of mice given a sodium thiopental i.p. administration (30 mg/kg) 20 min after an i.p. injection of vehicle or different doses of Hesperidin. The sleeping time was measured as the time spent between disappearance and reappearance of righting reflex. *p <0.05, ** p <0.001, different from vehicle, Dunn's comparison test after Kruskal-Wallis nonparametric ANOVA test. | | | |

Table 10 clearly shows that hesperidin is hypnotic.

### Effect on different behavioural tests in mice

All tests were performed as described in Example 5.

The results are shown in Table 11 expressed as percentage of decrements relative to vehicle values.

**Table 11 - Effectiveness of Valeriana and Citrus (Sigma) hesperidin on different behavioural tests in mice.**

| **Sample** | **Dose (mg/kg)** | **n** | **Locomotor Activity** | | **Hole-board test** | | | **Wire** |
|---|---|---|---|---|---|---|---|---|
| | | | **Total** | **ambulatory** | **N° of Head- dips** | **Time head dipping** | **N° of rearings** | |
| VEH | - | 11 | 100 | 100 | 100 | 100 | 100 | 0 |
| Valeriana hesperidin | 4 | 13 | **-55**** | **-58**** | **-74**** | **-81##** | **-58**** | 0 |
| Citrus hesperidin | 4 | 6 | -31 | -30 | -40 | -44 | -22 | 0 |

| | | | | | | | | |
|---|---|---|---|---|---|---|---|---|
| ** p< 0.01, Dunnet's after ANOVA. ## p < 0.001, Dunns's, non parametric. | | | | | | | | |

The results in Table 11 show that the citrus hesperidin is less active (w/w) than the Valeriana hesperidin. These *in vivo* results are in line with the analysis of the same samples performed by CD. that indicate that Citrus hesperidin contains app 60 % of pharmacologically available S active form. Table 11 therefore shows the sedative effect of hesperidin, including a clear depressant effect on locomotor activity.

### Example 11 - Potentiation of the effect of hesperidin on sleeping time in mice by simultaneous administration of 6-methylapigenin

This assay was carried out as described in Example 6. The results shown in Table 12 indicate that the sleep inducing effect of hesperidin is strongly potentiated by 6-methylapigenin. This finding is the first experimental evidence of the long-time held view of synergistic effects probably occurring in Valeriana.

**Table 12 - Sleeping time assay: potentiation of the effects of hesperidin by 6-methylapigenin.**

| **Sample (Dose)** | **n** | **Sleeping time Median (s)** |
|---|---|---|
| Vehicle | 15 | 0 |
| 6-methylapigenin (1 mg/kg) | 8 | 0 |
| Hesperidin Valeriana (2 mg/kg) | 16 | 179* |
| Hesperidin Valeriana (2 mg/kg) + 6-methylapigenin (1 mg/kg) | 10 | 1710** |

| | | |
|---|---|---|
| Median (interquartile range) of sleeping time of mice given a sodium thiopental i.p. administration (30 mg/kg) 20 min after an i.p. injection of vehicle or different drugs. The sleeping time was measured as the time spent between disappearance and reappearance of righting reflex. *p <0.05, ** p <0.001, different from vehicle, Dunn's comparison test after Kruskal-Wallis nonparametric ANOVA test. | | |

### Example 12 - Accounting of the in vivo activity of Valeriana wallichii crude extracts in terms of the amounts of hesperidin and 6-methylapigenin present in them

From 40 kg of *Valeriana wallichii* powder (Example 7), was recovered approximately 10 mg of pure 6-methylapigenin. Assuming a 0.1 to 1 % final recovery, the original concentration of 6-methylapigenin in the dried roots and rhizomes is approximately in the range of 25-250 µg/g.

In several experiments the yield of pure hesperidin was 50 mg per 100 g of dry *Valeriana wallichii* roots and rhizomes. Assuming a 10 % final yield, due to the rather simple procedure of extraction (see Example 9) we arrive at the final value of 5 mg/g for the concentration of hesperidin in the dry drug.

**Table 13 - Effectiveness on the sleeping time assay in mice of a fraction obtained in the isolation of hesperidin.**

| **Sample** | **Dose (mg/kg of mice)** | **n** | **Sleeping time Median (min)** |
|---|---|---|---|
| Vehicle | - | 6 | 0.2 |
| Aqueous remaining solution (**Table 3**) | 163* | 6 | 19 |
| Hesperidin | 4 | 7 | 20 |

| | | | |
|---|---|---|---|
| * According to the data in Table 3, 163 mg of Aqueous remaining solution (Fraction B) is equivalent to 1 g of dry drug which means that it contains approximately 4 mg of hesperidin. | | | |

The data in Table 13 supports the conclusion that the concentration of hesperidin in the *Valeriana wallichii* powder explains its sleep inducing activity. This conclusion is reinforced by the presumable synergistic action of 6-methylapigenin.

### Example 13 - Presence of 6-methylapigenin in V. officinalis L.

Powdered roots and rhizomes of *V*. *officinalis L*. were submitted to the fractionation scheme shown in Figure 2. The ethyl ether extract material was fractionated by HPLC as described in Example 1. The fractions were submitted to binding assays to detect activity on the BDZ-bs. The results shown in Table 14 indicate the presence of a ligand for this receptor only in Fraction D, which has the same elution volume as the one containing 6-methylapigenin (See Figure 1a).

**Table 14 - Results of receptor binding assays carried out as described in Example 2. Relatively low/high activity are indicated as + and +++, respectively and zero activity as -.**

| **Receptors activity** | **Fractions/binding level** | | | | | | | |
|---|---|---|---|---|---|---|---|---|
| | **A** | **B** | **C** | **D** | **E** | **F** | **G** | **H** |
| BDZ | - | - | - | +++ | + | - | - | - |

### Example 14 - Identification of hesperidin in V. officinalis L.

Powdered dry roots and rhizomes (50 g) of V. officinalis were suspended in 250mL of 70 % ethanol and the mixture was kept 2.5 hr at 37°C, with stirring. The filtrate was concentrated to 1/3 of the original volume to eliminate most of the ethanol and extracted with an equal volume of petroleum ether, that was discarded. The aqueous phase was extracted 3 times with an equal volume of amyl alcohol and the alcohol phase was evaporated to dryness. The solid residue dissolved in choloroform was chromatographed in a silica gel column as described in Example 9 to obtain a whitish residue that was collected and hydrolyzed in boiling 1 M HC1. The hydrolyzate was extracted with ethyl ether. The ether residue was submitted to HPLC (same conditions as in Figure 1) and the fraction eluting with the elution volume of hesperetin (the aglycone of hesperidin) was collected. This material when analyzed by mass spectroscopy (recorded on a Shimadzu Mass-Spectrometer QP-5000 at 70 eV with direct probe inlet) gave the following result: EIMS m/z 302 (M+), 285, 259, 179, 150. This spectrum was identical to one of authentic hesperetin obtained under the same conditions from hesperidin (Sigma).

This confirms the presence of hesperidin in *V*. *officinalis L*.

### Example 15 - Effect of the amyl alcohol extract from V officinalis, as shown in Example 14, on the sleeping time in mice.

The assay was carried out as described in Example 6.

**Table 15 - Effect of V. officinalis amyl alcohol extract on sleeping time.**

| **Sample (i.p.)** | **Dose** | **N** | **Sleeping Time Median (s)** |
|---|---|---|---|
| Vehicle | - | 8 | 0 |
| *Valeriana officinalis* | # | 10 | 510** |
| 2S-hesperidin | 3 | 9 | 500** |

| | | | |
|---|---|---|---|
| #Amount of amyl alcohol extract equivalent to 4 g of crude drug per kg. Median (interquartile range) of sleeping time of mice given a sodium thiopental i.p. administration (30 mg/kg) 20 min after an i.p. injection of vehicle or sample. The sleeping time was measured as the time spent between disappearance and reappearance of righting reflex. ** p <0.001, different from vehicle. Dunn's comparison test after Kruskal-Wallis nonparametric ANOVA test. | | | |

The results in Table 15 indicate a hypnotic activity in V. officinalis, in terms of hesperidin content, approximately ¼ of that found in *V*. *wallichii* (3 mg of hesperidin are equivalent to approximately 1 g of crude V. wallichii). (However, this estimate may have to be corrected for the probable existence of potentiating effects due to the presence of 6-methylapigenin in the *Valeriana officinalis* extract and also to the presence of linarin (see Example 17).

### Example 16 - Identification of Linarin in V. officinalis

In the fractionation procedure described in Example 14 the whitish precipitate obtained from the silica-gel column chromatography permitted the identification of the hesperidin that it contained. However, the HPLC (performed as in Example 1) of this material indicated the presence of a second component later identified as: 7-[[6-O-(6-deoxy-α-L-mannopyranosyl)-β-D-glucopyranosyl]oxy]-5-hydroxy-2-(4-methoxyphenyl)-4H-benzopyran-4-one or acacetin-β-rutinoside or linarin, besides hesperidin. The elution volumes were clearly different: for hesperidin: 11.7 mL; and for linarin : 15.3 mL.

Linarin was purified by crystallization from ethanolwater and identified by UV, ¹H-NMR and mass spectroscopy.

(Linarin is commonly known as 7-[[6-O-(6-deoxy-alpha-L-mannopyranosyl)-beta-D-glucopyranosyl]oxy]-5-hydroxy-2-(4-methoxyphenyl)-4H-benzopyran-4-one or acacetin-beta-rutinoside.

### Identification of Linarin

Linarin: acacetin-β-rutinoside: white sphero crystals from ethanol. UV λₘₐₓ (methanol): 327.5, 269.0, 210.0nm. ¹H NMR δ_{H} 12.90 (1H, s, OH-5), 8.05 (2H, d, J= 8.81 Hz, H-2' and H-6'), 7.14 (2H, d, J=9.10 Hz, H-3' and H-5'), 6.94 (1H, s, H-3), 6.78 (1H, d, J=2.06 Hz, H-8), 6.44 (1H, d, J= 2.06 Hz, H-6), 5.05 (1H, d, J= 7.04 Hz, H-1 glucosyl) , 4.44 (1H, d, J= 6.16 Hz, H-1 rhamnosyl), 3.86 (3H, s, OCH₃-4'), 3.09-3.46 (m, H-sugars), 1.07 (3H, d, J= 6.16 Hz, CH₃-rhamnosyl).

### Identification of Acacetin in Linarin

A sample of linarin was hydrolyzed with boiling 1 M HCl, for 1 hour. The aglycone, acacetin, was extracted from the hydrolysate with ethyl ether. The ether extract residue was submitted to ¹H NMR analysis.

Acacetin: λₘₐₓ 328.5, 268.0, 213.0nm. ¹H-NMR δ_{H} 12.91 (1H, s, OH-5), 10.84 (1H, s, OH-7), 8.03 (2H, d, J= 8.80 Hz, H-2' and H-6'), 7.10 (2H, d, J= 9.10 Hz, H-3' and H-5'), 6.86 (1H, s, H-3), 6.49 (1H, d, J= 2.05 Hz, H-8), 6.18 (1H, d, J= 2.05 Hz, H-6), 3.84 (3H, s, CH₃-4'). EMS m/z: 284 (M⁺), 269, 256, 241, 152.

Linarin was first identified in *V. wallichii* in 1968 (Thies, P. W. (1968). Linarinisovalerianate, ein bischer unbekannte Flavonoid aus V. wallichii D.C. *Planta medica,* **16**: 361-371), but its pharmacological properties were not explored. The present invention includes the first identification of linarin in *V*. *officinalis* and the discovery of its sedative and hypnotic activities.

### Example 17 - Effect of linarin on the sleeping time test in mice.

The assay was carried out as described in Example 6. The results are shown in Table 16 from which it may be estimated that the hypnotic activity of linarin is evident at the dose of 7 mg/kg.

**Table 16 - Effectiveness of linarin on the sleeping time assay in mice.**

| **Sample** | **Dose (mg/kg of mice)** | **n** | **Sleeping time Median (min)** |
|---|---|---|---|
| Vehicle | - | 16 | 0 |
| Linarin | 4 | 6 | 0 |
| | 7 | 5 | 30** |
| | 14 | 5 | 30** |

| | | | |
|---|---|---|---|
| Median (interquartile range) of sleeping time of mice given a sodium thiopental i.p. administration (30 mg/kg) 20 min after an i.p. injection of vehicle or different drugs. The sleeping time was measured as the time spent between disappearance and reappearance of righting reflex. ** p <0.001, different from vehicle, Dunn's comparison test after Kruskal-Wallis nonparametric ANOVA test. | | | |

### Example 18 - Potentiation of the effects of linarin on the sleeping time test in mice by simultaneous administration of valerenic acid.

This assay was carried out as described in Example 6.

The results shown in Table 17 indicate that the sleep inducing effect of linarin is strongly potentiated by valerenic acid. This synergistic effect is similar to that described in Example 11 for hesperidin and 6-methylapigenin.

**Table 17 - Sleeping time assay: potentiation of the effects of linarin by valerenic acid.**

| **Sample (Dose)** | **n** | **Sleeping time Median (min)** |
|---|---|---|
| Vehicle | 16 | 0 |
| Valerenic acid (5 mg/kg) | 6 | 0 |
| Valerenic acid (15mg/kg) | 5 | 0 |
| Linarin (4 mg/kg) | 6 | 0 |
| Linarin (4 mg/kg) + Valerenic acid (5 mg/kg) | 10 | 25** |

| | | |
|---|---|---|
| Median (interquartile range) of sleeping time of mice given a sodium thiopental i.p. administration (30 mg/kg) 20 min after an i.p. injection of vehicle or different drugs. The sleeping time was measured as the time spent between disappearance and reappearance of righting reflex. ** p <0.001, different from vehicle, Dunn's comparison test after Kruskal-Wallis nonparametric ANOVA test. Valerenic acid does not potentiate 6-methylapigenin, as shown in the following experiment | | |

**Table 18 - Sleeping time assay: lack of potentiation of the effects of Valerenic acid**

| **Sample (Dose)** | **n** | **Sleeping time Median (min)** |
|---|---|---|
| Vehicle | 6 | 0 |
| Valerenic acid (5 mg/kg) | 6 | 0 |
| 6-methylapigenin (1mg/kg) | 6 | 0 |
| 6-methylapigenin (1 mg/kg) + Valerenic acid (5 mg/kg) | 4 | 0 |

### Example 19 -Effects of linarin and valerenic acid on the Hole-board test in mice.

The test was carried out as described in Example 5. The results are shown in Table 19 expressed as percentage of decrements relative to vehicle values (see Table 4).

**Table 19 - Hole-board test assay: preliminary results of the effects of linarin and valerenic acid.**

| **Sample (dose)** | **n** | **Hole-board test** | | |
|---|---|---|---|---|
| | | **N° of Head-dips** | **Time head-dipping** | **N° of rearings** |
| VEH | 4 | 100 | 100 | 100 |
| Linarin (4 mg/Kg) + Valerenic acid (5 mg/Kg) | 5 | -35* | -47.4** | -63.2* |

| | | | | |
|---|---|---|---|---|
| * p<0.02, ** p<0.005, Unpaired Student's t-test | | | | |

The results in Table 19 show that linarin plus valerenic acid at the doses of 4 mg/kg and 5 mg/kg, respectively, are clearly sedative.

### Example 20 - Standardization of crude extracts (nutraceuticals) of V. officinalis and V. wallichii.

As described in the present invention, the sedative and sleep inducing properties of Valeriana species, in particular *V. officinalis* and *V. wallichii*, are predominantly due to their content of the flavonoids hesperidin, linarin and 6-methylapigenin plus in the case of *V*. *officinalis,* of the already known component of the essential oil; valerenic acid.

In the present invention we claim that all four mentioned components must be detected and quantitatively measured in the pharmaceutical preparations of valeriana so as to insure the provision of effective doses for human use. Accordingly the extracts selected to be used as, for example, nutraceuticals, should be submitted to the HPLC fractionation described in Example 1 and the area of the peaks of hesperidin and linarin (in *V*. *wallichii* and *V*. *officinalis* extracts), hesperidin, linarin and valerenic acid (in *V*. *officinalis* extracts) are integrated, and using as reference the standard curves of the pure compounds their quantification may be achieved in the usual way.

For this purpose the extracts selected to be used as nutraceuticals, should be submitted to the HPLC fractionation described in Example 1 and the area of the peaks of hesperidin (in *V*. *wallichii* and *V*. *officinalis* extracts), or linarin and valerenic acid (in *V*. *officinalis* extracts) are integrated, and using as reference the standard curves of the pure compounds their quantification may be achieved in the usual way.

6-Methylapigenin is measured in the same extracts described before, after their fractionation by HPLC, as in Example 1. 6-Methylapigenin is present in fraction D which is used to perform quantitative binding assays as described in Figure 6.

### Example 21 - Experimental data on apigenin and chrysin

Shown below in Table 20 is the results of apigenin and chrysin which are other flavanoids.

**Table 20**

| **Sample (Dose)** | **n** | **Sleeping time Median (min)** |
|---|---|---|
| Vehicle | 6 | 0 |
| Apigenin (1 mg/kg) | 4 | 0 |
| Chrysin (1mg/kg) | 6 | 0 |
| Hesperidin (2 mg/kg) | 15 | 3.5* |
| Hesperidin (2 mg/kg) + Apigenin (1mg/kg) | 12 | 10** |
| Hesperidin (2 mg/kg) + Chrysin (1 mg/kg) | 6 | 1.5 |

| | | |
|---|---|---|
| Median (interquartile range) of sleeping time of mice given a sodium thiopental i.p. administration (30mg/kg) 20 min after an i.p. injection of vehicle or the drugs. The sleeping time was measured as the time spent between disappearance and reappearance of righting reflex. *p < 0.05, **p <0.01, different from vehicle, Dunn's comparison test after Kruskal-Wallis nonparametric ANOVA test. | | |

Table 20 shows that apigenin and chrysin themselves do not produce any sleeping time i.e. there is no hypnotic activity. Hesperidin at 2mg/kg provides a sleeping time of 3.5min and therefore has a small hypnotic effect. Apigenin is also shown to have a potentiating effect on hesperidin due to the increase in sleeping time. However, chrysin is shown to inhibit the hypnotic activity of hesperidin due to the reduction in sleeping time. Table 20 therefore shows that not all flavanoids provide a potentiating effect.

### REFERENCES

(1) Medina, J. H.; Paladini A. C.; Wolfman, C.; Levi, M.; Calvo, D.; Diaz, L.; Peña, C. (1990) Chrysin (5,7 di-OH flavone) a naturally-occurring ligand for benzodiazepine receptors, with anticonvulsant properties. Biochem. Pharmacol., 40: 2227-2232.
(2) Nénonéné, E. K.; Radja, F.; Carli, M.; Grondin, L. y Reader,T. A. (1994). Heterogeneity of cortical and hippocampal 5-HT1A receptors: a reappraisal of homogenate binding with 8-[3H] hydroxydipropylaminotetralin. J. Neurochem., 62: 1822.
(3) Reyes-Parada, M.; Scorza, M. C.; Romero, M. V.; Silveira, R.; Medina, J. H.; Andrews, D.; Nichols, D. E.; Cassels, B. K. (1996). 1-(2,5-dimethoxy-4-ethyltiophenyl)-2-aminopropane (Aleph-2) a novel putative anxiolytic agent lacking affinity for the benzodiazepine sites and serotonin 1A receptor. Naunyn Scheiedeberg Arch. Pharmacol. 354: 579-585.
(4) Cammarota, M.; Bernabeu, R.; Levi de Stein, M.; Izquierdo I.; Medina, J. H. (1997). learning-specific, time-dependent increases in hippocampal CAMKII activity and AMPA GluR1 subunit immunoreactivity. Eur. J. Neurosc. 10: 2669-2676.
(5) El-Ani, D; Jacobson, K. A.; Shainberg, A. (1997). Regulation of A1 adenosine receptors by amiodarone and electrical stimulation in rat myocardial cells in vitro. Biochem. Pharmacol. 54: 583-587.
(6) File, S. E.; Pellow, S. (1985). The effects of triazolobenzodiazepines in two animal tests of anxiety and in the holeboard. Brit. J. Pharmacol. 86: 729-735.
(7) Wolfman, C., Viola, H., Marder, M., Wasowski, C., Ardenghi, P., Izquierdo, I., Paladini, A. C., Medina J. H. (1996) Anxioselective properties of 6,3'-dinitroflavone, a high affinity benzodiazepine receptor ligand. Europ. J. Pharm. 318: 23-30.
(8) Braestrup C., Honore T., Nielsen M., Petersen E. N., Jensen L. H. (1984). Ligands for benzodiazepine receptors with positive and negative efficacy. Biochem. Pharmacol.; 33: 859-862.
(9) Harborne, J. B. (1984) Phytochemical Methods (2nd Ed.), Chapman and Hall. London-New York.
(10) Higby, R. H. (1943). The chemical nature of Vitamin P. J. Am. Pharm. Assoc.; 32: 74-77.

## Claims

1. A process for obtaining an active component capable of inducing sedation in mammals from *valeriana* roots by a process comprising the steps of:
(a) 70%v/v aqueous ethanol extraction of comminuted valerian roots;
(b) 50%v/v aqueous ethyl ether extract of the ethanolic extract after removal of the ethanol to produce an ether extract first portion and a remaining aqueous portion;
(c) dissolving in a polar and/or non-polar solvent the ether extract first portion after removal of the ether;
(d) fractionating by chromatography the dissolved first portion by stepwise elution with one or more polar and/or non-polar solvents over a suitable solid phase; and
(e) recovering various fractions or taking the remaining aqueous fraction and testing for sedation benzodiazepine binging site (BDZ-bs) activity.

2. A process according to claim 1 wherein after removal of the ether in step (c), the ether extract first portion is dissolved in methanol prior to fractionation.

3. A process according to any of claims 1 or 2 wherein step (d) comprises the step of fractionating the dissolved first portion in a 10-80%v/v stepwise concentration gradient of aqueous acetonitrile using a C18 Vydac (Trade Name) reverse solid phase column, followed by step (f) comprising recovering a 40-50%v/v aqueous acetonitrile fraction displaying sedation activity.

4. A process according to any preceding claim wherein step (e) comprises *in vivo* and/or *in vitro* testing of the sedation and sleep activities of the various fractions and/or remaining aqueous portions.

5. A process according to any preceding claim which provides an active sedative component comprising 6-methylapigenin and hesperidin.

6. A process according to claim 5 wherein the 6-methylapigenin is present in the ethyl ether extract first portion.

7. A process according to any preceding claim wherein the active component is substantially free of at least one of the following compounds: valepotriates and baldrinals.

8. A process according to any preceding claim wherein the active component has high levels of activity in relation to both *in vivo* sedation activity and *in vitro* benzodiazepine receptor or binding site (BDZ-bs) binding activity.

9. Use of the active component obtainable according to any preceding claim for the preparation of a medicament for inducing sleep in 30g body weight mice at a dosage of the order of 100µg of active component (corresponding to a dosage of the order of 3000µg/kg body weight).

10. Use of 6-methylapigenin in the manufacture of a medicament for the preparation of an anxiolytic for mammals.

11. Active components capable of inducing sedation, sleep and/or anxiolysis in mammals obtainable from *valeriana* roots by a process comprising the steps of:
(a) about 70%v/v aqueous ethanol extraction of comminuted valerian roots;
(b) petroleum ether extraction of the aqueous solution remaining after the elimination of the ethanol by evaporation, the petroleum ether extract is then discarded;
(c) about 50%v/v aqueous ethyl ether extraction of the aqueous solution remaining in (b) after removal of the petroleum ether by evaporation, to produce an ethyl ether extract first portion and a remaining aqueous portion;
(d) dissolving in a polar and/or non-polar solvent the dry ether extract first portion after removal of the ether;
(e) fractionating by chromatography the dissolved ether extract first portion by stepwise elution with one or more polar and/or non-polar solvents over a suitable solid phase, and recovering various fractions to obtain a novel crystalline active material;
(f) extracting the aqueous phase remaining after the ethyl ether extraction with amyl alcohol and chromatographing this extract in a suitable solid phase column eluted with chloroform/methanol; and
(g) the fractions containing the active material in (f), detected by a sleep inducing assay, are pooled and partially evaporated to obtain a second novel crystalline active material.

12. A process according to claim 11 wherein step (e) comprises fractionating the dissolved ether extract first portion in a 10-80%v/v stepwise concentration aqueous acetonitrile gradient using a C18 Vydac (Trade Name) reverse solid phase column recovering a 40-50%v/v aqueous acetonitrile eluted fraction displaying sedative, sleep inducing or anxiolytic activity.

13. A process according to any of claims 11 or 12 wherein steps (e), (f) and/or (g) comprise *in vivo* and/or *in vitro* testing of the receptor binding and sedative, sleep and/or anxiolytic activities of the various fractions from the ether extract first portion and/or remaining aqueous portion.

14. A process according to any of claims 11 to 13 wherein the valeriana roots are *Valeriana wallichii* roots.

15. A process according to any of claims 11 to 14 wherein the novel active component obtained in step (f) is hesperidin.

16. A process according to claim 15 wherein the hesperidin is the S(-) form of hesperidin.

17. A process according to any of claims 11 to 16 wherein the novel active component obtained in step (e) is 6-methylapigenin.

18. A process according to claim 17 wherein the 6-methylapigenin is present in the ethyl extract first portion.

19. A process according to any of claims 11 to 13 or 15 to 17 wherein the valeriana roots used are *Valeriana officinalis.*

20. A process according to claim 19 wherein in step (f) linarin is obtained as a novel active component.

21. A process according to any of claims 11 to 20 wherein in step (f) a silica-gel column is used.

22. A process according to any of claims 11 to 21 wherein the fraction obtained from the ether extract has high levels of activity in relation to *in vitro* benzodiazepine binding site (BDZ-bs) binding activity and *in vivo* anxiolysis activity.

23. A process according to any of claims 11 to 22 wherein the aqueous remaining portion after the petroleum ether and ethyl ether extraction is used directly as a sedative dietary supplement.

24. A process according to claim 23 wherein the remaining aqueous portion comprises hesperidin and linarin.

25. A process according to claim 24 wherein the hesperidin is S(-) hesperidin.

26. Use of the active component obtainable according to any of claims 11 to 23 for the preparation of a medicament for use as a sedative, sleep inducing and/or anxiolytic agent.

27. Use according to claim 26 for cancelling anxiety, inducing sleep and/or acting as a sedative in mice and other species at a dosage of the order of 100µg of active component (corresponding to a dosage of the order of 3000µg/kg body weight).

28. An active sedative, sleep inducing and/or anxiolytic component comprising 6-methylapigenin.

29. Use of S(-) hesperidin, 6-methylapigenin, linarin or a combination thereof for the manufacture of a medicament for use in sedation in an animal, such as mammals, including humans.

30. Use of S(-) hesperidin, 6-methylapigenin, linarin or a combination for the manufacture of a medicament for use in tranquilizing in an animal, such as mammals, including humans.

31. Use of S(-) hesperidin, 6-methylapigenin, linarin or a combination thereof for the manufacture of a medicament for use in sleep induction in an animal, such as mammals, including humans.

32. Use according to any one of claims 29 - 31, which additionally includes use of an anxiolytic compound.

33. Use according to claim 32, wherein the anxiolytic compound is a flavonoid compound.

34. Use according to any of claims 29 to 33 wherein S(-) hesperidin and 6-methylapigenin are used in combination, and the 6-methylapigenin is found to have a potentiating and/or synergistic effect.

35. Use according to any of claims 29 to 33 wherein when linarin is used in combination with valerenic acid the valerenic acid is found to have a potentiating and/or synergistic effect.

36. A sedative, anxiolytic and/or sleep inducing dietary supplement comprising an active component according to any of claims 11 to 25 substantially free of at least one of the following compounds: valepotriates and baldrinals.

## Patentansprüche

1. Verfahren zum Erhalten einer aktiven Komponente aus Valeriana-Wurzeln, welche Komponente in der Lage ist, in Säugern eine Sedierung zu induzieren und welches Verfahren die Schritte umfasst:
(a) Extraktion von zerkleinerten Valeriana-Wurzeln mit 70%igem (Volumen/Volumen) wässrigen Ethanol;
(b) Extraktion des etbanoliscben Extraktes mit 50%igem (Volumen/Volumen) wässrigem Ethylether nach Entfernung des Ethanols zur Erzeugung eines ersten Ether-Extraktteils und eines verbleibenden wässrigen Teils;
(c) Auflösen des ersten Ether-Extraktteils in einem polaren und/oder unpolaren Lösemittel nach Entfernung des Ethers;
(d) Fraktionieren des aufgelösten ersten Teils mit Hilfe der Chromatographie durch schrittweise Elution mit einem oder mehreren polaren und/oder unpolaren Lösemitteln über einer geeigneten festen Phase;
(e) Gewinnen verschiedener Fraktionen oder Aufnehmen der zurückbleibenden wässrigen Fraktion und Testen auf Sedierungs- Benzodiazepin-Bindungsstellen (BDZ-bs: "benzodiazepine binding site")-Wirksamkeit.

2. Verfahren nach Anspruch 1, bei welchem nach Entfernung des Ethers in Schritt (c) der erste Ether-Extraktteil vor der Fraktionierung in Methanol aufgelöst wird.

3. Verfahren nach einem der Ansprüche 1 oder 2, wobei Schritt (d) den Schritt der Fraktionierung des aufgelösten ersten Teils in einem 10% bis 80%igem (Volumen/Volumen) schrittweisen Konzentrationsgradienten von wässrigem Acetonitril umfasst, indem eine C18-Vydac (Warenzeichen)-Umkehrfestphasensäule verwendet wird, gefolgt von Schritt (f), der das Gewinnen einer 40% bis 50%igen (Volumen/Volumen) wässrigen Acetonitril-Fraktion umfasst, die Sedierungswirksamkeit zeigt.

4. Verfahren nach einem der vorgenannten Ansprüche, wobei Schritt (e) ein *in vivo*- und/oder *in vitro*-Testen der Sedierungs- und Schlafwirksamkeiten der verschiedenen Fraktionen oder verbleibenden wässrigen Teile umfasst.

5. Verfahren nach einem der vorgenannten Ansprüche, welches Verfahren eine aktive sedierende Komponente bereitstellt, die 6-Methylapigenin und Hesperidin aufweist.

6. Verfahren nach Anspruch 5, wobei das 6-Methylapigenin in dem ersten Ethylether-Extraktteil vorliegt.

7. Verfahren nach einem der vorgenannten Ansprüche, wobei die aktive Komponente weitgehend frei ist von mindestens einer der folgenden Verbindungen: Valepotriate und Baldrinale.

8. Verfahren nach einem der vorgenannten Ansprüche, wobei die aktive Komponente über eine hochgradige Wirksamkeit in Bezug auf sowahl *in vivo*-Sedierungswirksamkeit als auch *in vitro-*Benzodiazepinrezeptor- oder -Bindungsstellen (BDZ-bs)-Bindungswirksamkeit verfügt

9. Verwendung der aktiven Komponente, die nach einem der vorgenannten Ansprüche erhalten werden kann, für die Herstellung eines Medikaments zum Auslösen von Schlaf in Mäusen mit 30 g Körpergewicht bei einer Dosierung in der Größenordnung von 100 µg aktive Komponente (entsprechend einer Dosierung in der Größenordnung von 3.000 µg/kg Körpergewicht).

10. Verwendung von 6-Methylapigenin in der Herstellung eines Medikaments für das Präparieren eines Anxiolytikums für Säuger.

11. Aktive Komponenten mit der Fähigkeit zum Induzieren von Sedierung, von Schlaf und/oder Anxiolyse in Säugern, die erhalten werden können aus Valeriana-Wurzeln mit Hilfe eines Verfahrens, das die Schritte umfasst:
(a) Extraktion von zerkleinerten Valeriana-Wurzeln mit etwa 70%igem (Volumen/Volumen) wässrigen Ethanol;
(b) Petrotether-Extraktion der wässrigen Lösung, die nach der Eliminierung des Ethanols durch Abdampfen verbleibt, wobei der Petrolether-Extrakt anschließend verworfen wird;
(c) etwa 50%ige (Volumen/Volumen) wässrige Ethylether-Extraktion der wässrigen Lösung, die in (b) nach der Entfernung des Petrolethers durch Abdampfen zurückbleibt, um einen ersten Ethylether-Extraktteil und einen verbleibenden wässrigen Teil zu erzeugen:
(d) Auflösen des wasserfreien ersten Ether-Extraktteils in einem polaren und/oder unpolaren Lösemittel nach Entfernung des Ethers;
(e) Fraktionieren des aufgelösten ersten Ether-Extrakteils mit Hilfe der Chromatographie durch schrittweise Elution mit einem oder mehreren polaren und/oder unpolaren Lösemitteln über einer geeigneten festen Phase und Gewinnen verschiedener Fraktionen, um ein neuartiges kristallines aktives Material zu erhalten.
(f) Extrahieren der nach der Ethylether-Extraktion zurückbleibenden wässrigen Phase mit Amylalkohol sowie Chromatographieren deses Extraktes in einer geeigneten Festphasensäule, die mit Chloroform/Methanol eluiert wird; und
(g) die Fraktionen, die das aktive Material in (f) enthalten, die mit Hilfe eines Schlaf induzierenden Assays detektiert wurden, werden gepoolt und teilweise eingedampft, um ein zweites, neuartiges kristallines aktives Material zu erhalten.

12. Verfahren nach Anspruch 11, wobei Schritt (e) das Fraktionieren des aufgelösten ersten Ether-Extraktteils in einer schrittweisen 10-80% Volumen/Volumen Konzentrationsgradienten von wässrigem Acetonitril umfasst, indem eine C18-Vydac (Warenzeichen)-Umkehrfestphasensäule verwendet wird, die eine 40% bis 50%ige (Volumen/Volumen) mit wässrigem Acetonitril eluierte Fraktion gewinnt, welche sedierende, Schlaf auslösende oder anxiolytische Wirksamkeit zeigt.

13. Verfahren nach einem der Ansprüche 11 oder 12, bei welchem die Schritte (e), (f) und/oder (g) das *in vivo*- und/oder *in vitro*-Testen der rezeptorbindenden und sedierenden, Schlaf- und/oder anxiolytischen Wirksamkeiten der verschiedenen Fraktionen aus dem ersten Ether-Extraktteil und/oder verbleibenden wässrigen Teil umfassen.

14. Verfahren nach einem der Ansprüche 11 bis 13, bei welchem die Valeriana-Wurzeln Wurzeln der *Valeriana wallichi* sind.

15. Verfahren nach einem der Ansprüche 11 bis 14, bei welchem die neuartige aktive Komponente, die in Schritt (f) erhalten wird, Hesperidin ist.

16. Verfahren nach Anspruch 15, wobei das Hesperidin die S(-)-Form des Hesperidins ist.

17. Verfahren nach einem der Ansprüche 11 bis 16, bei welchem die neuartige aktive Komponente, die in Schritt (e) erhalten wird, 6-Methylapigenin ist.

18. Verfahren nach Anspruch 17, wobei das 6-Methylapigenin in dem ersten Ethyl-Extraktteil anwesend ist.

19. Verfahren nach einem der Ansprüche 11 bis 13 oder 15 bis 17, bei welchem die zur Anwendung gelangenden Valeriana-Wurzeln *Valeriana officinalis* ist.

20. Verfahren nach Anspruch 19, bei welchem in Schritt (f) als neuartige Komponente Linarin erhalten wird.

21. Verfahren nach einem der Ansprüche 11 bis 20, bei welchem in Schritt (f) eine Silicagel-Säule zur Anwendung gelangt.

22. Verfahren nach einem der Ansprüche 11 bis 21, bei welchem die Fraktion, die aus dem Ether-Extrakt erhalten wird, über eine hochgradige Wirksamkeit in Bezug auf *in vitro*-Benzodiazepin-Bindungsstellen (BDZ-bs)-bindende Wirksamkeit und über eine *in vivo*-Anxiolysewirksamkeit verfügt.

23. Verfahren nach einem der Ansprüche 11 bis 22, bei welchem der nach der Petrolether- und Ethylether-Extraktion verbleibende wässrige Teil direkt als eine sedierende Nahrungsmittelergänzung verwendet wird.

24. Verfahren nach Anspruch 23, wobei der verbleibende wässrige Teil Hesperidin und Linarin aufweist.

25. Verfahren nach Anspruch 24, wobei Hesperidin S(-)-Hesperidin ist.

26. Verwendung der aktiven Komponente, die nach einem der Ansprüche 11 bis 23 erhalten werden kann, für die Herstellung eines Medikaments zur Verwendung als ein sedierendes, Schlaf auslösendes und/oder anxiolytisches Mittel.

27. Verwendung nach Anspruch 26 zum Lösen von Angst, zum Auslösen von Schlaf und/oder zur Wirkung als ein Sedativum bei Mäusen und anderen Spezies bei Dosierung in der Größenordnung von 100 µg aktive Komponente (entsprechend einer Dosierung in der Größenordnung von 3.000 µg/kg Körpergewicht).

28. Aktive sedierende, Schlaf auslösende und/oder anxiolytische Komponente, die 6-Methylapigenin aufweist.

29. Verwendung von S(-)-Hesperidin, 6-Methylapigenin, Linarin oder einer Kombination davon für die Herstellung eines Medikaments zur Verwendung in der Sedierung eines Tieres, wie beispielsweise eines Säugers, einschließlich Menschen.

30. Verwendung von S(-)-Hesperidin, 6-Methylapigenin, Linarin oder einer Kombination davon für die Herstellung eines Medikaments zur Verwendung in der Beruhigung eines Tieres, wie beispielsweise eines Säugers, einschließlich Menschen.

31. Verwendung von S(-)-Hesperidin, 6-Methylapigenin, Linarin oder einer Kombination davon für die Herstellung eines Medikaments zur Verwendung in der Schlafauslösung eines Tieres, wie beispielsweise eines Säugers, einschließlich Menschen.

32. Verwendung nach einem der Ansprüche 29 bis 31, die zusätzlich die Verwendung einer anxiolytischen Verbindung einschließt,

33. Verwendung nach Anspruch 32, wobei die anxiolytische Verbindung eine Flavanaid-Verbindung ist.

34. Verwendung nach einem der Ansprüche 29 bis 33, wobei das S(-)-Hesperidin und 6-Methylapigenin in Kombination verwendet werden und von dem 6-Methylapigenin festgestellt wurde, dass es eine potenzierende und/oder synergistische Wirkung hat.

35. Verwendung nach einem der Ansprüche 29 bis 33, wobei festgestellt worden ist, dass, wenn Linarin in Kombination mit Valerensäure verwendet wird, die Valerenensäure eine potenzierende und/oder synergistische Wirkung hat.

36. Sedierende, anxiolytische und/oder Schlaf auslösende sedierende Nahrungsmittelergänzung, aufweisend eine aktive Komponente nach einem der Ansprüche 11 bis 25, die weitgehend frei ist von mindestens einer der folgenden Verbindungen: Valepotriate und Baldrinale.

## Revendications

1. Procédé d'obtention d'un composant actif capable d'induire la sédation chez les mammifères à partir de racines de *Valeriana* par un procédé comprenant les étapes de:
(a) extraction avec de l'éthanol aqueux à 701% vol/vol de racines de valériane finement broyées;
(b) extraction avec de l'éther d'éthyle aqueux à 50% vol/vol de l'extrait éthanolique après l'élimination de l'éthanol pour produire une première portion d'extrait à l'éther et une portion aqueuse restante;
(c) dissolution dans un solvant polaire et/ou non polaire de la première portion d'extrait à l'éther après l'élimination de l'éther;
(d) fractionnement par chromatographie de la première portion dissoute par élution par étape avec un ou plusieurs solvant(s) polaire(s) et/ou non polaire(s) sur une phase solide appropriée; et
(e) récupération des fractions variées ou prélèvement de la faction aqueuse restante et test de l'activité de sédation du site de liaison de benzodiazépine (BDZ-bs).

2. Procédé selon la revendication 1, dans lequel après l'élimination de l'éther à l'étape (c), la première portion d'extrait à l'éther est dissoute dans du méthanol avant le fractionnement.

3. Procédé selon l'une quelconque des revendications 1 ou 2, dans lequel l'étape (d) comprend l'étape de fractionnement de la première portion dissoute dans un gradient de concentration par étape de 10 à 80% vol/vol d'acétonitrile aqueux en utilisant une colonne en phase solide inverse C18 Vydac (appellation commerciale), suivie de l'étape (f) comprenant la récupération d'une fraction d'acétonitrile aqueux à 40 à 50 % vol/vol affichant une activité de sédation.

4. Procédé selon l'une quelconque des revendications précédentes, dans lequel l'étape (e) comprend le test *in vivo* et/ou *in vitro* d'activités de sédation et d'endormissement des fractions variées et/ou des portions aqueuses restantes.

5. Procédé selon l'une quelconque des revendications précédentes, qui fournit un composant sédatif actif comprenant de la 6-méthylapigénine et de l'hespéridine.

6. Procédé selon la revendication 5, dans lequel la 6-méthylapigénine est présente dans la première portion d'extrait à l'éther d'éthyle.

7. Procédé selon l'une quelconque des revendications précédentes, dans lequel le composant actif est substantiellement exempt d'au moins l'un des composés suivants: valépotriates et baldrinals.

8. Procédé selon l'une quelconque des revendications précédentes, dans lequel le composant actif présente des niveaux élevés d'activité en relation à la fois avec l'activité de sédation *in vivo* et l'activité de liaison *in vitro* au récepteur ou au site de liaison de benzodiazépine (BDZ-bs).

9. Utilisation du composant actif pouvant être obtenu selon l'une quelconque des revendications précédentes pour la préparation d'un médicament pour induire le sommeil chez des souris de 30 g de poids corporel à une dose de l'ordre de 100 µg de composant actif (correspondant à une dose de l'ordre de 3 000 µg/kg de poids corporel).

10. Utilisation de 6-méthylapigénine dans la fabrication d'un médicament pour la préparation d'un anxiolytique pour des mammifères.

11. Composants actifs capables d'induire la sédation, le sommeil et/ou l'anxiolyse chez des mammiferes pouvant être obtenus à partir de racines de *Valeriana* par un procédé comprenant les étapes de:
(a) extraction à l'éthanol aqueux à environ 70 % vol/vol de racines de valériane finement broyées;
(b) extraction à l'éther de pétrole de la solution aqueuse restante après l'élimination de l'éthanol par évaporation, l'extrait d'éther de pétrole est ensuite jeté;
(c) extraction aqueuse à l'éther d'éthyle à environ 50% vol/vol de la solution aqueuse restante dans (b) après l'élimination de l'éther de pétrole par évaporation, pour produire une première portion d'extrait à l'éther d'éthyle et une portion aqueuse restante;
(d) dissolution dans un solvant polaire et/ou non polaire de la première portion d'extrait à l'éther sec après l'élimination de l'éther;
(e) fractionnement par chromatographie de la première portion d'extrait à l'éther dissous par élution par étape avec un ou plusieurs solvant(s) polaire(s) et/ou non polaire(s) sur une phase solide appropriée, et la récupération des fractions variées pour obtenir un nouveau matériau actif cristallin;
(f) extraction de la phase aqueuse restante après l'extraction à l'éther d'éthyle avec de l'alcool d'amyle et la chromatographie de cet extrait dans une colonne en phase solide appropriée éluée avec du chloroforme/méthanol; et
(g) les fractions contenant le matériau actif dans (f), détecté par un essai d'induction du sommeil, sont rassemblées et partiellement évaporées pour obtenir un second nouveau matériau actif cristallin.

12. Procédé selon la revendication 11, dans lequel l'étape (e) comprend le fractionnement de la première portion d'extrait à l'éther dissous dans un gradient de concentration par étape d'acétonitrile aqueux à 10 à 80% vol/vol en utilisant une colonne en phase solide inverse C18 Vydac (appellation commerciale) récupérant une fraction aqueuse éluée avec de l'acétonitrile à 40 à 50 % vol/vol affichant une activité sédative, d'induction du sommeil ou anxiolytique.

13. Procédé selon l'une quelconque des revendications 11 ou 12, dans lequel les étapes (e), (f) et/ou (g) comprennent un test *in vivo* et/ou *in vitro* d'activités de liaison à un récepteur et sédative, d'induction du sommeil et/ou anxiolytique des fractions variées à partir de la première portion d'extrait à l'éther et/ou de la portion aqueuse restante.

14. Procédé selon l'une quelconque des revendications 11 à 13, dans lequel les racines de valériane sont des racines de *Valeriana wallichii.*

15. Procédé selon l'une quelconque des revendications 11 à 14, dans lequel le nouveau composant actif obtenu dans l'étape (f) est l'hespéridine.

16. Procédé selon la revendication 15, dans lequel l'hespéridine est la forme S(-) de l'hespéridine.

17. Procédé selon l'une quelconque des revendications 11 à 16, dans lequel le nouveau composant actif obtenu à l'étape (e) est la 6-méthylapigénine.

18. Procédé selon la revendication 17, dans lequel la 6-méthylapigénine est présente dans la première portion d'extrait éthylique.

19. Procédé selon l'une quelconque des revendications 11 à 13 ou 15 à 11, dans lequel les racines de valériane utilisées sont de la *Valeriana officinalis*.

20. Procédé selon la revendication 19, dans lequel dans l'étape (f) de la linarine est obtenue comme nouveau composant actif.

21. Procédé selon l'une quelconque des revendications 11 à 20, dans lequel dans l'étape (f) une colonne de gel de silice est utilisée.

22. Procédé selon l'une quelconque des revendications 11 à 21, dans lequel la fraction obtenue à partir de l'extrait à l'éther présente des niveaux élevés d'activité en relation avec l'activité *in vitro* de liaison au site de liaison de benzodiazépine (BDZ-bs) et avec l'activité *in vivo* d'anxiolyse.

23. Procédé selon l'une quelconque des revendications 11 à 22, dans lequel la portion aqueuse restante après l'extraction avec de l'éther de pétrole et de l'éther d'éthyle est utilisée directement comme complément alimentaire sédatif.

24. Procédé selon la revendication 23, dans lequel la portion aqueuse restante comprend de l'hespéridine et de la linarine.

25. Procédé selon la revendication 24, dans lequel l'hespéridine est la S(-) hespéridine.

26. Utilisation du composant actif pouvant être obtenu selon l'une quelconque des revendications 11 à 23 pour la préparation d'un médicament pour l'utilisation comme agent sédatif, d'induction du sommeil et/ou anxiolytique.

27. Utilisation selon la revendication 26 pour éliminer l'anxiété, induire le sommeil et/ou agir comme sédatif chez les souris et d'autres espèces à une dose de l'ordre de 100µg de composant actif (correspondant à une dose de l'ordre de 3 000 µg/kg de poids corporel).

28. Composant actif sédatif, d'induction du sommeil et/ou anxiolytique comprenant de la 6-méthylapigénine.

29. Utilisation de la S(-) hespéridine, 6-méthylapigénine, linarine ou d'une combinaison de celles-ci pour la fabrication d'un médicament pour l'utilisation dans la sédation chez un animal, tel que les mammifères, incluant les êtres humains,

30. Utilisation de S(-) hespéridine, 6-méthylapigénine, linarine ou une combinaison pour la fabrication d'un médicament pour l'utilisation pour tranquilliser un animal, tel que des mammifères, incluant les êtres humains.

31. Utilisation de S(-) hespéridine, 6-méthylapigénine, linarine ou d'une combinaison de celles-ci pour la fabrication d'un médicament pour l'utilisation dans l'induction du sommeil chez un animal, tel que les mammifères, incluant les êtres humains.

32. Utilisation selon l'une quelconque des revendications 29 à 31, qui inclut de manière supplémentaire l'utilisation d'un composé anxiolytique.

33. Utilisation selon la revendication 32, dans laquelle le composé anxiolytique est un composé de type flavonoïde.

34. Utilisation selon l'une quelconque des revendications 29 à 33, dans laquelle la S(-) hespéridine et la 6-méthylapigénine sont utilisées en combinaison, et il est découvert que la 6-méthylapigénine possède un effet de potentialisation et/ou de synergie.

35. Utilisation selon l'une quelconque des revendications 29 à 33, dans laquelle lorsque de la linarine est utilisée en combinaison avec l'acide valérénique il est découvert que l'acide valérénique possède un effet de potentialisation et/ou de synergie.

36. Complémentaire alimentaire sédatif, anxiolytique et/ou d'induction du sommeil comprenant un composant actif selon l'une quelconque des revendications 11 à 25 substantiellement exempt d'au moins l'un des composés suivants: valépotriates et baldrinals.
